# EUROPEAN PATENT SPECIFICATION

(11) **EP 3 600 422 B1**
(45) Date of publication and mention of the grant of the patent: **18.10.2023**
(21) Application number: 18774300.0
(22) Date of filing: 30.03.2018
(51) Int. Cl.: A61K 39/395, C07K 14/435, C07K 16/28, C07K 16/46, A61P 35/00

(54) **COMPOSITIONS FOR USE IN METHODS FOR TARGETING AND KILLING ALPHA-V BETA-3-POSITIVE CANCER STEM CELLS (CSCS) AND TREATING DRUG RESISTANT CANCERS**
ZUSAMMENSETZUNGEN ZUR VERWENDUNG IN DEM VERFAHREN ZUM ABZIELEN UND TÖTEN VON ALPHA-V BETA-3-POSITIVEN KREBSSTAMMZELLEN (CSCS) UND IN DER BEHANDLUNG VON ARZNEIMITTELRESISTENTEN TUMOREN
COMPOSITIONS POUR L'UTILISATION DANS DES MÉTHODES DE CIBLAGE ET DE DESTRUCTION DE CELLULES SOUCHES CANCÉREUSES (CSC) POSITIVES À ALPHA-V BÊTA-3 ET DANS LE TRAITEMENT DE CANCERS PHARMACORÉSISTANTS

(30) Priority: 31.03.2017 US 201762479768 P
(43) Date of publication of application: 05.02.2020
(73) Proprietor: The Regents of the University of California, Oakland, CA 94607 (US)
(72) Inventor: CHERESH, David, La Jolla California 92093 (US); WETTERSTEN, Hiromi, La Jolla California 92093 (US)
(74) Representative: Cantaluppi, Stefano
(86) International application number: PCT/US2018/025470
(87) International publication number: WO 2018/183894

(56) References cited:
- WO-A1-03/075957
- WO-A1-2007/059195
- WO-A1-2013/152313
- WO-A1-2015/068847
- WO-A1-2016/100858
- US-A1- 2006 040 325
- US-A1- 2013 225 494
- Tae Jin Kim ET AL: "Combined anti-angiogenic therapy against VEGF and integrin alpha V beta 3 in an orthotopic model of ovarian cancer", , 1 January 2009 (2009-01-01), XP055754571, Retrieved from the Internet: URL:https://www.ncbi.nlm.nih.gov/pmc/artic les/PMC3400500/pdf/nihms310726.pdf [retrieved on 2020-11-27]

## Description

### RELATED APPLICATIONS

This Patent Convention Treaty (PCT) International Application claims the benefit of priority to U.S. Provisional Application No. 62/479,768, filed March 31, 2017.

### GOVERNMENT RIGHTS

This invention was made with government support under grant numbers T32 OD017853; T32 HL086344; T32 HL098062-03; R-857GC; NCI R01CA045726, awarded by the National Institutes of Health (NIH). The government has certain rights in the invention.

### TECHNICAL FIELD

This invention generally relates to immunology and oncology. Provided are compositions for use in treating or ameliorating a cancer by targeting cell surface-expressed αvβ3 (avb3) polypeptides in Cancer Stem Cells (CSCs) to kill the CSCs, thus treating ameliorating or slowing the development of cancers caused or initiated by or sustained by cancer or tumor cells, or Cancer Stem Cells (CSCs), expressing αvβ3 polypeptides on their cell surfaces.

### BACKGROUND

Tumor associated macrophages are involved in regulation of cancer growth and aggressiveness. Whereas M1 macrophages trigger an inflammatory response and inhibit tumor growth, M2 macrophages secrete pro-tumor cytokines into the microenvironment to support tumor progression. A macrophage switch from M1 to M2 has been associated with lung cancer progression, and cancer stem cells have been implicated as a driver of this reprogramming.

WO 2013/152313 discloses that erlotinib resistant cancer cells, tumor cells or cancer stem cells are treating by shRNA knocking down integren β3

WO 03/075957 describes antibody Vitaxin for use in the treatment of treatment-naive cancer including cancer expressing αvβ3, such as carcinomas.

The in vitro effectiveness of the combined treatment of paclitaxel and the integrin αvβ3 antibody etaracizumab in the taxane-resistant ovarian carcinoma SKOV3TRip2 cells expressing integrin αvβ3 is disclosed by Tae Jin Kim et al., ,2009, "Combined anti-angiogenic therapy against VEGF and integrin alpha Vbeta3 in an orthotopic model of ovarian cancer", Cancer Biology and Therapy, 8(23), 2263-2272.

### SUMMARY

The invention is as defined in the claims.

Provided are methods for
- killing or reducing the number of αvβ3 (avb3) polypeptide-expressing cancer cells or Cancer Stem Cells (CSCs) in an individual in need thereof,
   the methods comprising:
      (a) administering to an individual in need thereof an antibody capable of specifically binding to an αvβ3 (avb3) integrin polypeptide expressed on a cancer or a tumor cell, or on a CSC, or
      (b) (i) providing an antibody or polypeptide capable of specifically binding to an αvβ3 (avb3) integrin polypeptide expressed on a cancer or a tumor cell, or on a CSC,
   wherein the antibody or polypeptide has an Fc domain or equivalent domain or moiety capable of binding a macrophage and initiating an antibody-dependent cell-mediated cytotoxicity (ADCC) killing of the cell to which the antibody specifically binds
      (ii
- increasing a macrophage population capable of triggering an inflammatory response and inhibiting tumor growth *in vivo,* wherein optionally the macrophage population capable of triggering an inflammatory response and inhibiting tumor growth comprises a tumor associated macrophage (TAM) or an M1 macrophage population,
   wherein the antibody is: monoclonal antibody LM609 (Chemicon Int., Temecula, CA)

Provided are methods: wherein the macrophage is a human macrophage, or a tumor associated macrophage (TAM), an M1 macrophage, or a tumor-inhibiting M2 macrophage.

Provided are methods: wherein the cancer is an epithelial cancer or epithelial tumor cell; or wherein the cancer is a drug resistant cancer, and optionally the drug is a growth factor inhibitor or a kinase inhibitor, wherein optionally the growth factor inhibitor comprises a Receptor Tyrosine Kinase (RTK) inhibitor, optionally erlotinib.

Provided are methods: wherein the antibody is administered intravenously, intramuscularly or subcutaneously to the individual in need thereof; or wherein the antibody is formulated as a sterile pharmaceutical composition or formulation, or is formulated for administration intravenously, intramuscularly or subcutaneously; or wherein the dosage of antibody is based on either fixed or body weight-based dosing, or a fixed dose of between about 100 and 1200 mg monthly, or at a dosage of between about 0.3 to 10 mg/kg.

Provided are methods further comprising administration of a growth factor inhibitor, wherein optionally the growth factor inhibitor comprises a Receptor Tyrosine Kinase (RTK) inhibitor, a Src inhibitor, an anti-metabolite inhibitor, a gemcitabine, a GEMZAR^{™}, a mitotic poison, a paclitaxel, a taxol, an ABRAXANE^{™}, an erlotinib, a TARCEVA^{™}, a lapatinib, a TYKERB^{™}, a cetuxamib, an ERBITUX^{™}, or an insulin growth factor inhibitor.

The details of one or more exemplary embodiments are set forth in the accompanying drawings and the description below. Other features, objects, and advantages of the invention will be apparent from the description and drawings, and from the claims.

### BRIEF DESCRIPTION OF THE DRAWINGS

The drawings set forth herein are illustrative of embodiments as provided herein.
FIG. 1A-I schematically and graphically illustrates evaluation of the pathway of acquired resistance involving lung cancer resistance to EGFR inhibitors, which leads to the upregulation of αvβ3 and the acquisition of a drug resistant stem-like fate:
FIG. 1A: schematically illustrates a schematic of the erlotinib resistant xenograft model, i.e., how mice bearing αvβ3-negative EGFR-mutant human non-small cell lung cancer (HCC827) subcutaneous xenografts were treated with vehicle or erlotinib, and measured tumor growth over time;
FIG. 1B: graphically illustrates data showing how tumors eventually began to re-grow after several weeks, showing tumor volume as a function of days treated, as compared to control ("Veh", or vehicle), HCC827 (β3-) xenograft mice were treated with control or erlotinib;
FIG. 1C: illustrates an image of tumor tissues were stained for β3;
FIG. 1D: graphically illustrates data from a flow cytometry study where cells isolated from erlotinib resistant tissues were stained for αvβ3 and ALDH1A1;
FIG. 1E: graphically illustrates data from a flow cytometry where tumor cells isolated from a vehicle treated mouse (veh) and an erlotinib treated mouse (ErlR) were stained for αvβ3;
FIG. 1F: graphically illustrates cell viability after cells were treated with erlotinib or osimertinib for 72 hours;
FIG. 1G: graphically illustrates data showing that erlotinib increased CTCs while the primary tumors are still responding to the treatment; where 8 weeks after lung injection of HCC827 (β3-, GFP+, luciferase+) cells, the mice were treated with erlotinib for 30 days before CTCs isolation, and the CTCs were stained for αvβ3, and. αvβ3-positive (β3+) and -negative (β3-) CTCs from mice before (pre-treatment, n=3) and after the treatment (post-treatment, n=5) were counted;
FIG. 1H: illustrates an image of the primary masses of FIG. 1G;
FIG. 1I: graphically illustrates data showing how M2-TAMs were increased in the elrotinib resistant xenograft tissues; percentage of M1 (dark grey), and M2-TAMs (light grey)) in the tumor tissues was compared between the vehicle (n=9) and erlotinib (n=9) treatments after 50 days of the treatments;
as discussed in further detail in Example 1, below.
FIG. 2A-E schematically and graphically illustrates data showing that LM609 produced a less aggressive phenotype:

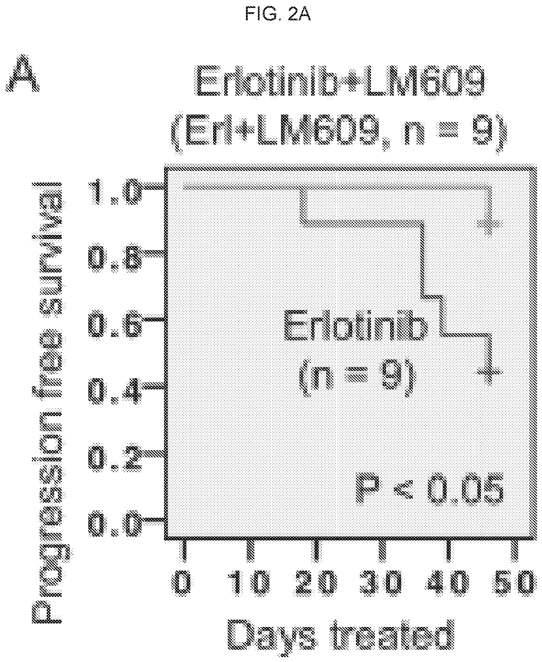

FIG. 2A graphically illustrates LM609 inhibited acquisition of erlotinib resistance, where HCC827 (β3-) xenograft mice were treated with erlotinib or erlotinib and LM609;

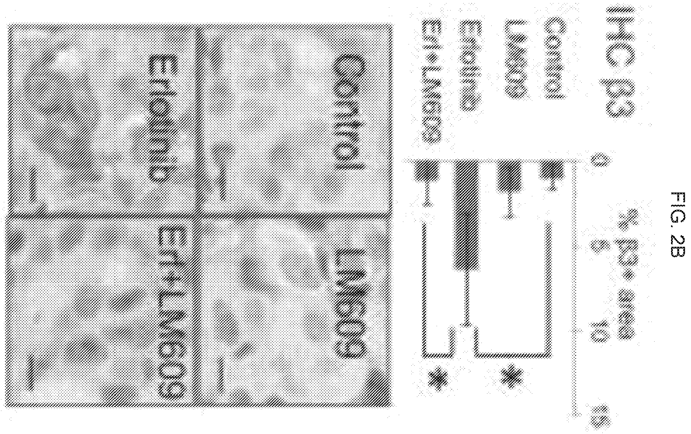

FIG. 2B upper and lower panels graphically and in images illustrate data and stained tissue images showing that LM609 eliminated β3-positive cells; where HCC827 (β3-) xenograft mice were treated with Captisol and PBS, Captisol and LM609, erlotinib and PBS, or erlotinib and LM609 for 50 days, and tumor tissues were stained (lower panel) for integrin β3, and β3-positive area in the tissues were quantified (upper panel);

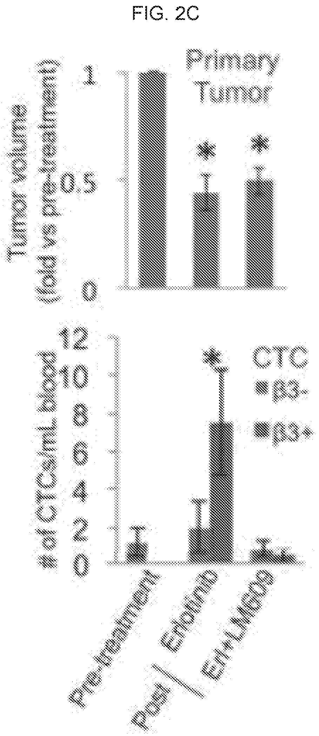

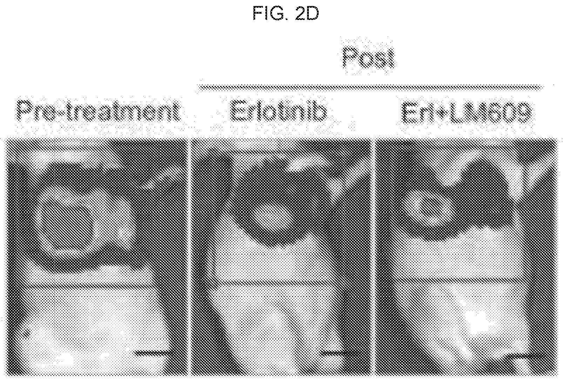

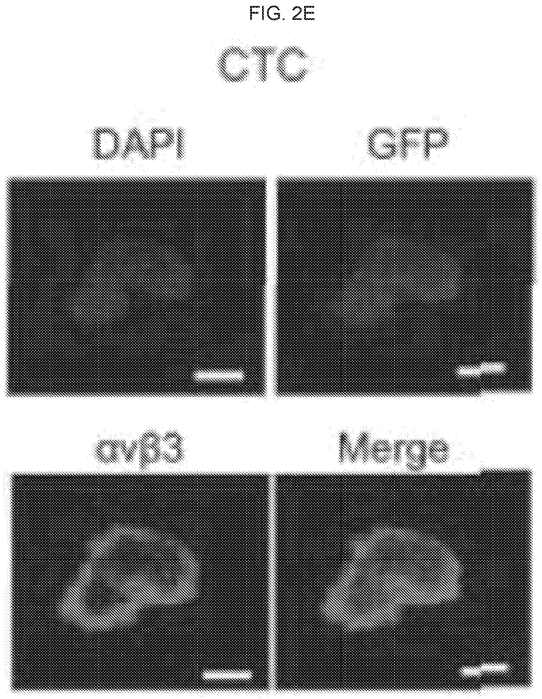

FIG. 2C-E schematically and graphically illustrate that LM609 decreased numbers of CTCs induced by erlotinib: eight weeks after the right lung injection of HCC827 (β3-, GFP+, luciferase+) cells, the mice were treated with erlotinib or the combination of erlotinib and LM609 (erlotinib+LM609) for four weeks before CTCs were isolated, and tumor volume was measured by bioluminescence (FIG. 2D) before the treatments and after the treatment with erlotinib (Post-erlotinib, n=4) or the combination of erlotinib and LM609 (Post-Erl+LM609, n=5) and quantified (FIG. 2C, upper panel); CTCs were stained for αvβ3 to quantify αvβ3-positive (β3+) and - negative (β3-) CTCs (FIG. 2C, lower panel); an example of αvβ3-positive CTCs is shown (FIG. 2E);
as discussed in further detail in Example 1, below.

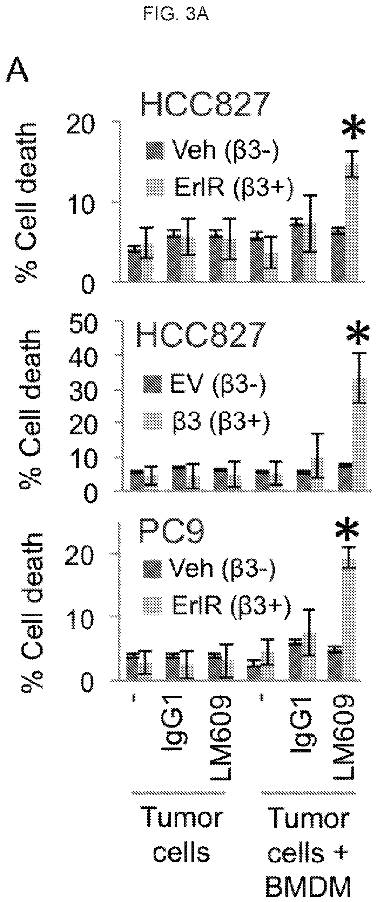

FIG. 3A-E graphically illustrate that LM609 eliminated αvβ3-positive cells *via* macrophage-mediated ADCC:
   FIG. 3A-C: LM609 eliminated αvβ3-positive cells *via* macrophage-mediated ADCC *in vitro;* ADCC assays with bone marrow derived macrophages (BMDMs) or NK cells were performed with cancer cells with and without αvβ3 integrin treated with the IgG isotype or LM609 10 µg/mL and/or Fc blocker;

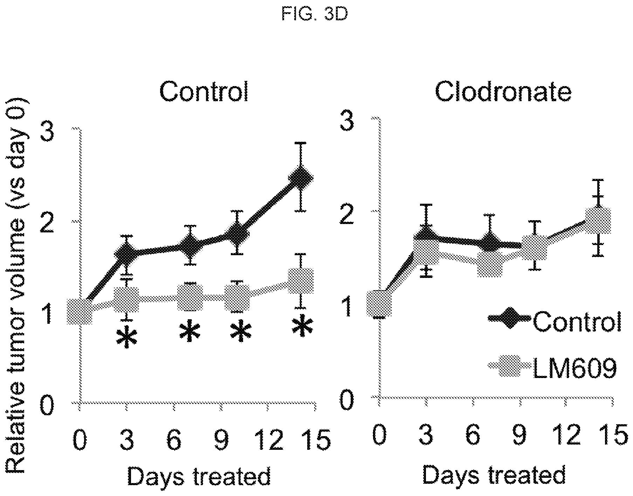

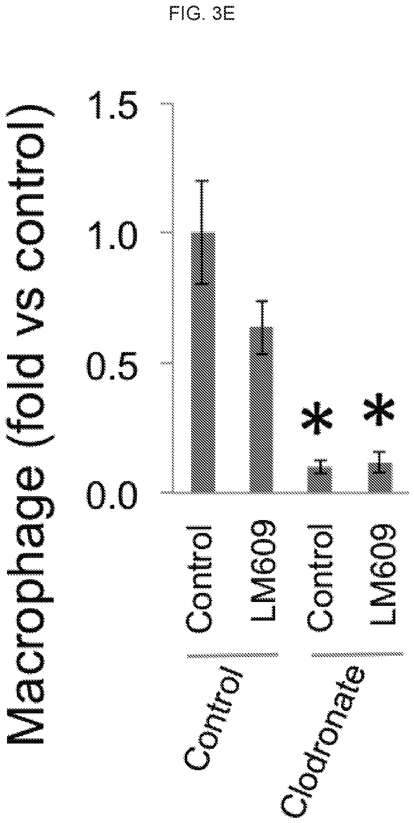

   FIG. 3D-E: LM609 eliminated αvβ3-positive tumor growth only in the mice that have macrophages. Nude mice were subcutaneously injected with β3 ectopically expressing HCC827 cells and treated with control liposome and PBS (FIG. 3D left panel, control), control liposome and LM609 (FIG. 3D left panel, LM609), clodronate liposome and PBS (FIG. 3D right panel, control), or clodronate liposome and LM609 (FIG. 3D right panel, LM609), tumor growth was monitored for 15 days, and after the treatments, F4/80 staining in the tumor tissues was quantified (FIG. 3E);
   as discussed in further detail in Example 1, below.

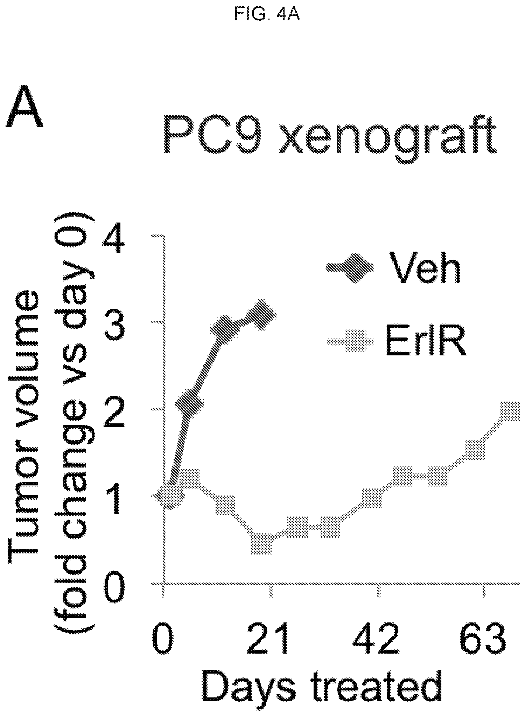

   FIG. 4A-C graphically illustrate that erlotinib resistant tumor cells gained αvβ3 integrin and were resistant to osimertinib:
      FIG. 4A graphically illustrate data showing that erlotinib treated tumor gained resistance, where PC9 (β3-) subcutaneous xenograft mice were treated with the vehicle (Veh) (n=1) or erlotinib (ErlR, n=1);

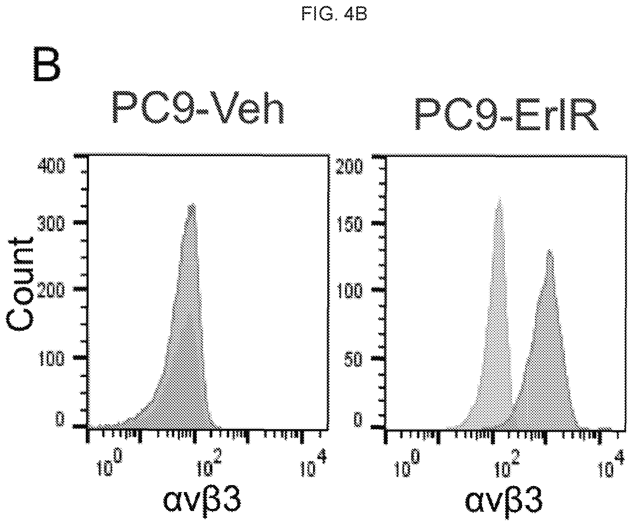

      FIG. 4B graphically illustrate data showing cells from erlotinib resistant tissue were αvβ3-positive while the cells from the vehicle treated animal were not; levels of αvβ3 on cells isolated from the vehicle treated (Veh, left panel) and erlotinib treated (ErlR, right panel) animals were measured by flow cytometry;

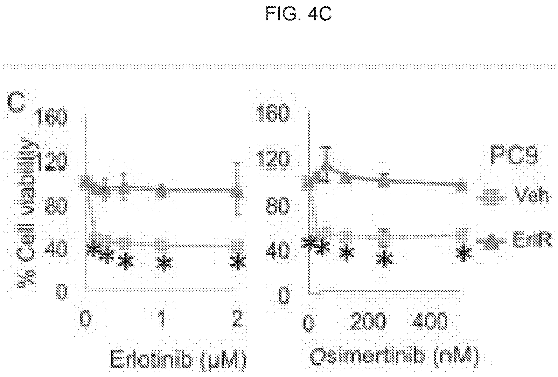

      FIG. 4C: Erlotinib resistant cells were osimertinib resistant. Cells from the vehicle treated (Veh) and erlotinib treated (ErlR) animals were treated with erlotinib (left panel) or osimertinib (right panel) at the indicated doses and MTT assay was performed to measure cell viability;
      as discussed in further detail in Example 1, below.

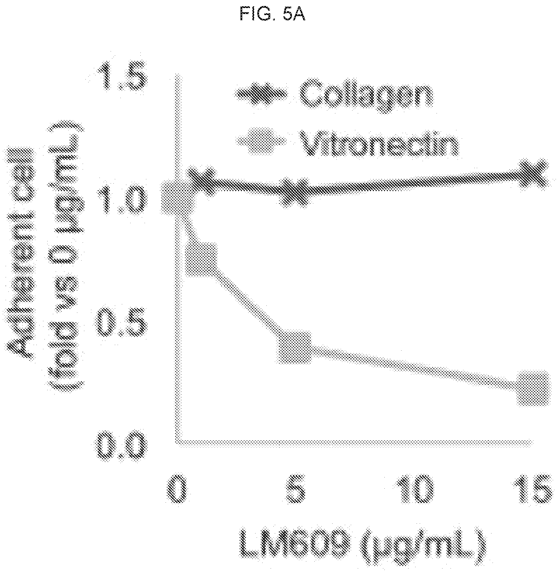

      FIG. 5A-B graphically illustrate data showing LM609 inhibited ligand binding of αvβ3 integrin but not cell viability:
         FIG 5A graphically illustrates data showing that LM609 inhibited ligand binding of integrin αvβ3, wherein M21 cells (αvβ3+) were plated on collagen or vitronectin (αvβ3 ligand) with indicated doses of LM609, and the adherent cell number was measured;

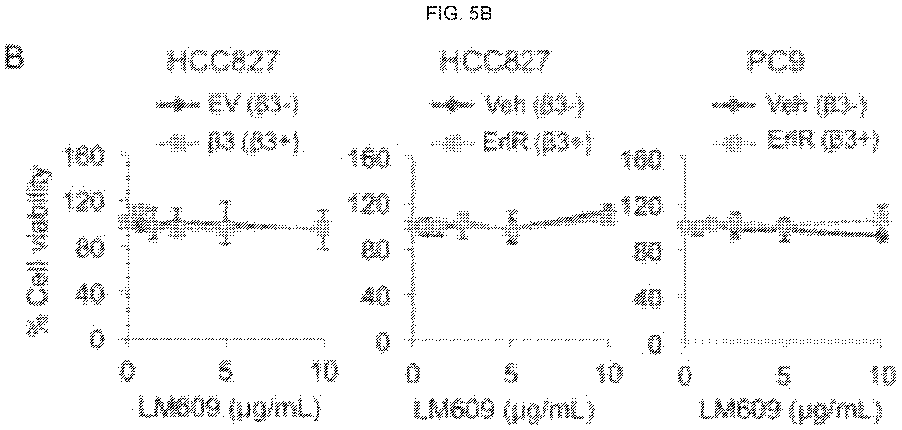

         FIG. 5B graphically illustrates data showing that LM609 did not affect cell viability, paired αvβ3-positive (β3+) and -negative (β3-) cells were treated with indicated doses of LM609, and MTT assay was performed to measure cell viability.

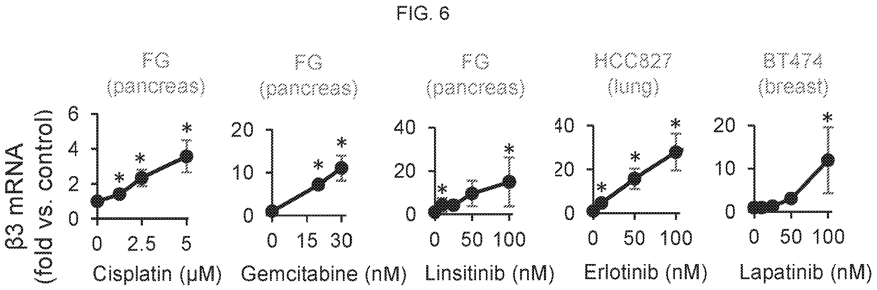

         FIG. 6 graphically illustrates data showing that cancer therapeutics enrich β3 integrin in epithelial cancer cells: qPCR was performed to detect mRNA expression of integrin α and β subunits in response to increasing dose of hydrogen peroxide (H2O2) for 72h, expressed as fold relative to vehicle control.

Like reference symbols in the various drawings indicate like elements.

Reference will now be made in detail to various exemplary embodiments, examples of which are illustrated in the accompanying drawings. The following detailed description is provided to give the reader a better understanding of certain details of aspects and embodiments as provided herein.

### DETAILED DESCRIPTION

The invention is as defined in the claims.

Provided are compositions and methods for treating or ameliorating a cancer by targeting cell surface-expressed αvβ3 (avb3) polypeptides in Cancer Stem Cells (CSCs) to kill the CSCs, thus treating ameliorating or slowing the development of cancers caused or initiated by or sustained by cancer or tumor cells, or Cancer Stem Cells (CSCs), expressing αvβ3 polypeptides on their cell surfaces. Provided are compositions and methods for targeting and killing αvβ3-positive Cancer Stem Cells (CSCs) and treating drug resistant cancers. Compositions and methods as provided herein use an antibody that can specifically bind to human αvβ3 that also comprises an Fc portion that can mediate antibody-dependent cell-mediated cytotoxicity (ADCC) killing of cancer or tumor cells by macrophages; use a humanized antibody to αvβ3 that has been modified to include an engineered Fc portion that specifically binds to human macrophages. Administration of the anti-avb3 antibody can eradicate or reduce the number of highly aggressive, αvβ3-positive drug resistant cancers by recruiting tumor associated macrophages which bind to the antibody (which also binds to αvβ3), and the macrophages thus kill the cancer by ADCC. Humanized antibodies such as the anti αvβ3 antibody LM609, are used to target αvβ3 polypeptides *in vivo* to bind the αvβ3-positive cancer cell, e.g., a CSC, and by also binding to macrophages, kill CSCs expressing the αvβ3 polypeptide by ADCC.

Anti-αvβ3 antibody LM609 (Chemicon Int., Temecula, CA); CBL544, derived from clone 23C6 (MilliporeSigma, Burlington, MA); ab7166 (abcam, Cambridge, MA); and, monoclonal antibody ab78289 (abcam, Cambridge, MA), and humanized versions thereof, is used to prolong cancer drug sensitivity in individuals in need thereof by targeting drug resistant cancer cells via macrophage mediated antibody dependent cell mediated cytotoxicity (ADCC).

Compositions and methods as provided herein are used to target and kill Cancer Stem Cells (CSCs), which can represent the most aggressive and drug resistant cells within a tumor population. Compositions and methods as provided herein are used to target integrin αvβ3, a polypeptide which identifies a CSC population within various epithelial cancers and is both necessary and sufficient to promote drug resistance.

The anti-αvβ3 integrin antibody, LM609, prolonged sensitivity to erlotinib in an EGFR mutant lung adenocarcinoma xenograft model. Described herein are studies showing that treatment of lung tumor bearing mice with the EGFR inhibitor, erlotinib, while initially suppressing tumor growth, ultimately results in tumor associated αvβ3 expression, leading to tumor progression as measured by an increase in circulating tumor cells (CTCs). In addition, erlotinib treated mice showed a marked accumulation of tumor-associated macrophages (TAMs) relative to untreated tumors. Systemic delivery of LM609, a monoclonal antibody directed to αvβ3, destroys the drug resistant CSCs within the primary tumor and eliminates CTCs. This anti-tumor effect is macrophage dependent as mice depleted of macrophages are unable to respond to this antibody. *In vitro,* LM609 in combination with macrophages but not NK cells destroy CSCs due to antibody dependent cellular cytotoxicity (ADCC). These findings demonstrate that erlotinib, while initially controlling tumor growth, ultimately leads to increased sternness, drug resistance, tumor progression and accumulation of TAMs. Evidence is provided that LM609, by targeting αvβ3, can direct TAMs to destroy the most drug resistant cells within the primary tumor, thereby controlling tumor progression.

We recently reported that integrin αvβ3 expression is induced on lung adenocarcinoma cells during drug resistance and is both necessary and sufficient to reprogram these tumors to a stem-like state. Given the role that cancer stem cells play in switching M1 to M2 macrophages (macrophage switch from M1 to M2 has been associated with lung cancer progression), we asked whether αvβ3 expression on lung adenocarcinoma cells account for this macrophage conversion. The M1/M2 macrophage ratio in αvβ3-positive tumors was markedly decreased relative to tumors lacking αvβ3. We next treated mice bearing αvβ3-positive tumors with a monoclonal antibody (LM609) targeting this receptor to assess its ability to alter the macrophage phenotype within these tumors. LM609 was able to selectively eliminate the αvβ3-positive cancer stem cells via antibody-dependent cell-mediated cytotoxicity (ADCC), markedly enhancing the sensitivity of these tumors to the effects of therapy. These findings revealed that αvβ3-expressing cancer stem cells enrich the pro-tumor M2 tumor-associated macrophages (TAMs). Eliminating αvβ3-positive cancer stem cells via ADCC serves to prolong tumor sensitivity to therapy.

### Pharmaceutical Compositions and Formulations

Provided are pharmaceutical compositions and formulations, e.g., comprising anti- αvβ3 (avb3) antibodies, and methods for: killing or reducing the number of αvβ3 (avb3) polypeptide-expressing cancer cells or Cancer Stem Cells (CSCs) in an individual in need thereof, treating, ameliorating or reversing or slowing the development of an αvβ3 (avb3) polypeptide-expressing cancer or tumor in an individual in need thereof, ameliorating or slowing the development of cancers caused or initiated by or sustained by cancer or tumor cells, or Cancer Stem Cells (CSCs), expressing αvβ3 polypeptides on their cell surfaces, manipulating TAMs *in vivo,* or enhancing the sensitivity of αvβ3 (avb3) polypeptide-expressing cancer or tumor to the effects of therapy.

Compositions provided herein, and compositions used to practice the methods provided herein, are formulated with a pharmaceutically acceptable carrier. In alternative embodiments, the pharmaceutical compositions used to practice the methods provided herein can be administered parenterally, topically, orally or by local administration, such as by aerosol or transdermally. The pharmaceutical compositions can be formulated in any way and can be administered in a variety of unit dosage forms depending upon the condition or disease and the degree of illness, the general medical condition of each patient, the resulting preferred method of administration and the like. Details on techniques for formulation and administration are well described in the scientific and patent literature, see, e.g., the latest edition of Remington's Pharmaceutical Sciences, Maack Publishing Co, Easton PA ("Remington's").

Therapeutic agents as provided herein, e.g., comprising anti- αvβ3 (avb3) antibodies, and antibodies used to practice methods as provided herein, can be administered alone or as a component of a pharmaceutical formulation (composition), or concurrently with, before and/or after administration with another active agent, e.g., a growth factor inhibitor, wherein optionally the growth factor inhibitor comprises a Receptor Tyrosine Kinase (RTK) inhibitor, a Src inhibitor, an anti-metabolite inhibitor, a gemcitabine, a GEMZAR^{™}, a mitotic poison, a paclitaxel, a taxol, an ABRAXANE^{™}, an erlotinib, a TARCEVA^{™}, a lapatinib, a TYKERB^{™}, a cetuxamib, an ERBITUX^{™}, or an insulin growth factor inhibitor.

Pharmaceutical compositions and formulations, e.g., comprising anti- αvβ3 (avb3) antibodies, may be formulated for administration in any convenient way for use in human or veterinary medicine. Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Formulations of the compositions provided herein and as used to practice the methods provided herein include those suitable for oral/ nasal, topical, parenteral, rectal, and/or intravaginal administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect.

Pharmaceutical formulations provided herein and as used to practice the methods provided herein can be prepared according to any method known to the art for the manufacture of pharmaceuticals. Such drugs can contain sweetening agents, flavoring agents, coloring agents and preserving agents. A formulation can be admixtured with nontoxic pharmaceutically acceptable excipients which are suitable for manufacture. Formulations may comprise one or more diluents, emulsifiers, preservatives, buffers, excipients, etc. and may be provided in such forms as liquids, powders, emulsions, lyophilized powders, sprays, creams, lotions, controlled release formulations, tablets, pills, gels, on patches, in implants, etc.

Pharmaceutical formulations for oral administration can be formulated using pharmaceutically acceptable carriers well known in the art in appropriate and suitable dosages. Such carriers enable the pharmaceuticals to be formulated in unit dosage forms as tablets, geltabs, pills, powder, dragees, capsules, liquids, lozenges, gels, syrups, slurries, suspensions, etc., suitable for ingestion by the patient. Pharmaceutical preparations for oral use can be formulated as a solid excipient, optionally grinding a resulting mixture, and processing the mixture of granules, after adding suitable additional compounds, if desired, to obtain tablets or dragee cores. Suitable solid excipients are carbohydrate or protein fillers include, e.g., sugars, including lactose, sucrose, mannitol, or sorbitol; starch from corn, wheat, rice, potato, or other plants; cellulose such as methyl cellulose, hydroxypropylmethyl-cellulose, or sodium carboxy-methylcellulose; and gums including arabic and tragacanth; and proteins, e.g., gelatin and collagen. Disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound (i.e., dosage). Pharmaceutical preparations provided herein and as used to practice the methods provided herein can also be used orally using, e.g., push-fit capsules made of gelatin, as well as soft, sealed capsules made of gelatin and a coating such as glycerol or sorbitol. Push-fit capsules can contain active agents mixed with a filler or binders such as lactose or starches, lubricants such as talc or magnesium stearate, and, optionally, stabilizers. In soft capsules, the active agents can be dissolved or suspended in suitable liquids, such as fatty oils, liquid paraffin, or liquid polyethylene glycol with or without stabilizers.

Aqueous suspensions can contain an active agent (e.g., an anti- αvβ3 (avb3) antibody or polypeptide) in admixture with excipients suitable for the manufacture of aqueous suspensions. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Oil-based pharmaceuticals are particularly useful for administration hydrophobic active agents (e.g., an anti- αvβ3 (avb3) antibody or polypeptide) used to practice the methods provided herein. Oil-based suspensions can be formulated by suspending an active agent in a vegetable oil, such as arachis oil, olive oil, sesame oil or coconut oil, or in a mineral oil such as liquid paraffin; or a mixture of these. See e.g., U.S. Patent No. 5,716,928 describing using essential oils or essential oil components for increasing bioavailability and reducing inter- and intra-individual variability of orally administered hydrophobic pharmaceutical compounds (see also U.S. Patent No. 5,858,401). The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto (1997) J. Pharmacol. Exp. Ther. 281:93-102. The pharmaceutical formulations provided herein can also be in the form of oil-in-water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent.

In practicing embodiment provided herein, the pharmaceutical compounds can also be administered by in intranasal, intraocular and intravaginal routes including suppositories, insufflation, powders and aerosol formulations (for examples of steroid inhalants, see Rohatagi (1995) J. Clin. Pharmacol. 35:1187-1193; Tjwa (1995) Ann. Allergy Asthma Immunol. 75:107-111). Suppositories formulations can be prepared by mixing the drug with a suitable non-irritating excipient which is solid at ordinary temperatures but liquid at body temperatures and will therefore melt in the body to release the drug. Such materials are cocoa butter and polyethylene glycols.

In practicing embodiments provided herein, the pharmaceutical compounds can be delivered by transdermally, by a topical route, formulated as applicator sticks, solutions, suspensions, emulsions, gels, creams, ointments, pastes, jellies, paints, powders, and aerosols.

In practicing embodiments provided herein, the pharmaceutical compounds can also be delivered as microspheres for slow release in the body. For example, microspheres can be administered via intradermal injection of drug which slowly release subcutaneously; see Rao (1995) J. Biomater Sci. Polym. Ed. 7:623-645; as biodegradable and injectable gel formulations, see, e.g., Gao (1995) Pharm. Res. 12:857-863 (1995); or, as microspheres for oral administration, see, e.g., Eyles (1997) J. Pharm. Pharmacol. 49:669-674.

In practicing embodiments provided herein, the pharmaceutical compounds can be parenterally administered, such as by intravenous (IV) administration or administration into a body cavity or lumen of an organ. These formulations can comprise a solution of active agent dissolved in a pharmaceutically acceptable carrier. Acceptable vehicles and solvents that can be employed are water and Ringer's solution, an isotonic sodium chloride. In addition, sterile fixed oils can be employed as a solvent or suspending medium. For this purpose, any bland fixed oil can be employed including synthetic mono- or diglycerides. In addition, fatty acids such as oleic acid can likewise be used in the preparation of injectables. These solutions are sterile and generally free of undesirable matter. These formulations may be sterilized by conventional, well known sterilization techniques. The formulations may contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of active agent in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated using those suitable dispersing or wetting agents and suspending agents. The sterile injectable preparation can also be a suspension in a nontoxic parenterally-acceptable diluent or solvent, such as a solution of 1,3-butanediol. The administration can be by bolus or continuous infusion (e.g., substantially uninterrupted introduction into a blood vessel for a specified period of time).

The pharmaceutical compounds and formulations provided herein and as used to practice the methods provided herein can be lyophilized. Also provided are stable lyophilized formulations comprising a composition provided herein, which can be made by lyophilizing a solution comprising a pharmaceutical provided herein on and a bulking agent, e.g., mannitol, trehalose, raffinose, and sucrose or mixtures thereof. A process for preparing a stable lyophilized formulation can include lyophilizing a solution about 2.5 mg/mL protein, about 15 mg/mL sucrose, about 19 mg/mL NaCl, and a sodium citrate buffer having a pH greater than 5.5 but less than 6.5. See, e.g., U.S. patent app. no. 20040028670.

The compositions and formulations provided herein and as used to practice the methods provided herein can be delivered by the use of liposomes. By using liposomes, particularly where the liposome surface carries ligands specific for target cells, or are otherwise preferentially directed to a specific organ, one can focus the delivery of the active agent into target cells *in vivo.* See, e.g., U.S. Patent Nos. 6,063,400; 6,007,839; Al-Muhammed (1996) J. Microencapsul. 13:293-306; Chonn (1995) Curr. Opin. Biotechnol. 6:698-708; Ostro (1989) Am. J. Hosp. Pharm. 46:1576-1587.

The formulations provided herein and as used to practice the methods provided herein can be administered for prophylactic and/or therapeutic treatments. In therapeutic applications, compositions are administered to a subject already suffering from a condition, infection or disease in an amount sufficient to cure, alleviate or partially arrest the clinical manifestations of the condition, infection or disease and its complications (a "therapeutically effective amount"). For example, in alternative embodiments, pharmaceutical compositions provided herein are administered in an amount sufficient to: kill or reduce the number of αvβ3 (avb3) polypeptide-expressing cancer cells or Cancer Stem Cells (CSCs) in an individual in need thereof; treating, ameliorating or reversing or slowing the development of an αvβ3 (avb3) polypeptide-expressing cancer or tumor in an individual in need thereof; and/ or ameliorating or slowing the development of cancers caused or initiated by or sustained by cancer or tumor cells, or Cancer Stem Cells (CSCs), expressing αvβ3 polypeptides on their cell surfaces. The amount of pharmaceutical composition adequate to accomplish this is defined as a "therapeutically effective dose." The dosage schedule and amounts effective for this use, i.e., the "dosing regimen," will depend upon a variety of factors, including the stage of the disease or condition, the severity of the disease or condition, the general state of the patient's health, the patient's physical status, age and the like. In calculating the dosage regimen for a patient, the mode of administration also is taken into consideration.

The dosage regimen also takes into consideration pharmacokinetics parameters well known in the art, i.e., the active agents' rate of absorption, bioavailability, metabolism, clearance, and the like (see, e.g., Hidalgo-Aragones (1996) J. Steroid Biochem. Mol. Biol. 58:611-617; Groning (1996) Pharmazie 51:337-341; Fotherby (1996) Contraception 54:59-69; Johnson (1995) J. Pharm. Sci. 84:1144-1146; Rohatagi (1995) Pharmazie 50:610-613; Brophy (1983) Eur. J. Clin. Pharmacol. 24: 103-108; the latest Remington's, supra). The state of the art allows the clinician to determine the dosage regimen for each individual patient, active agent and disease or condition treated. Guidelines provided for similar compositions used as pharmaceuticals can be used as guidance to determine the dosage regiment, i.e., dose schedule and dosage levels, administered practicing the methods provided herein are correct and appropriate.

Single or multiple administrations of formulations can be given depending on the dosage and frequency as required and tolerated by the patient. The formulations should provide a sufficient quantity of active agent to effectively treat, prevent or ameliorate a conditions, diseases or symptoms as described herein. For example, an exemplary pharmaceutical formulation for oral administration of compositions provided herein or as used to practice the methods provided herein can be in a daily amount of between about 0.1 to 0.5 to about 20, 50, 100 or 1000 or more *u*g per kilogram of body weight per day. In an alternative embodiment, dosages are from about 1 mg to about 4 mg per kg of body weight per patient per day are used. Lower dosages can be used, in contrast to administration orally, into the blood stream, into a body cavity or into a lumen of an organ. Substantially higher dosages can be used in topical or oral administration or administering by powders, spray or inhalation. Actual methods for preparing parenterally or non-parenterally administrable formulations will be known or apparent to those skilled in the art and are described in more detail in such publications as Remington's, supra.

The methods provided herein can further comprise co-administration with other drugs or pharmaceuticals, e.g., compositions for treating cancer, septic shock, infection, fever, pain and related symptoms or conditions. For example, the methods and/or compositions and formulations provided herein can be co-formulated with and/or co-administered with antibiotics (e.g., antibacterial or bacteriostatic peptides or proteins), particularly those effective against gram negative bacteria, fluids, cytokines, immunoregulatory agents, anti-inflammatory agents, complement activating agents, such as peptides or proteins comprising collagen-like domains or fibrinogen-like domains (e.g., a ficolin), carbohydrate-binding domains, and the like and combinations thereof.

### Nanoparticles and Liposomes

Also provided are nanoparticles and liposomal membranes comprising compounds (e.g., anti- αvβ3 (avb3) antibodies) used to practice the methods provided herein. In alternative embodiments, also provided are nanoparticles and liposomal membranes targeting tumor (cancer) stem cells and dysfunctional stem cells. In one aspect, the compositions used to practice the methods provided herein are specifically targeted to cancer cells or cancer stem cells.

In alternative embodiments, also provided are nanoparticles and liposomal membranes comprising (in addition to comprising compounds used to practice the methods provided herein) molecules, e.g., peptides or antibodies, that selectively target abnormally growing, diseased, infected, dysfunctional and/or cancer (tumor) cell receptors. In alternative embodiments, also provided are nanoparticles and liposomal membranes using IL-11 receptor and/or the GRP78 receptor to targeted receptors on cells, e.g., on tumor cells, e.g., on prostate or ovarian cancer cells. See, e.g., U.S. patent application publication no. 20060239968.

Also provided are nanocells to allow the sequential delivery of two different therapeutic agents with different modes of action or different pharmacokinetics, at least one of which comprises a composition used to practice the methods provided herein. A nanocell is formed by encapsulating a nanocore with a first agent inside a lipid vesicle containing a second agent; see, e.g., Sengupta, et al., U.S. Pat. Pub. No. 20050266067. The agent in the outer lipid compartment is released first and may exert its effect before the agent in the nanocore is released. The nanocell delivery system may be formulated in any pharmaceutical composition for delivery to patients.

In one embodiment, an inhibitor or depleter of an acetyl transferase gene, transcript (message) and/or protein expression or activity is contained in the outer lipid vesicle of the nanocell, and an antiangiogenic agent provided herein is loaded into the nanocore. This arrangement allows active agents to be released first and delivered to the tumor before the tumor's blood supply is cut off by the composition provided herein.

Also provided are multilayered liposomes comprising compounds used to practice embodiments provided herein, e.g., for transdermal absorption, e.g., as described in Park, et al., U.S. Pat. Pub. No. 20070082042. The multilayered liposomes can be prepared using a mixture of oil-phase components comprising squalane, sterols, ceramides, neutral lipids or oils, fatty acids and lecithins, to about 200 to 5000 nm in particle size, to entrap a composition provided herein.

A multilayered liposome used to practice embodiments provided herein may further include an antiseptic, an antioxidant, a stabilizer, a thickener, and the like to improve stability. Synthetic and natural antiseptics can be used, e.g., in an amount of 0.01% to 20%. Antioxidants can be used, e.g., BHT, erysorbate, tocopherol, astaxanthin, vegetable flavonoid, and derivatives thereof, or a plant-derived antioxidizing substance. A stabilizer can be used to stabilize liposome structure, e.g., polyols and sugars. Exemplary polyols include butylene glycol, polyethylene glycol, propylene glycol, dipropylene glycol and ethyl carbitol; examples of sugars are trehalose, sucrose, mannitol, sorbitol and chitosan, or a monosaccharide or an oligosaccharide, or a high molecular weight starch. A thickener can be used for improving the dispersion stability of constructed liposomes in water, e.g., a natural thickener or an acrylamide, or a synthetic polymeric thickener. Exemplary thickeners include natural polymers, such as acacia gum, xanthan gum, gellan gum, locust bean gum and starch, cellulose derivatives, such as hydroxy ethylcellulose, hydroxypropyl cellulose and carboxymethyl cellulose, synthetic polymers, such as polyacrylic acid, poly-acrylamide or polyvinylpyrollidone and polyvinylalcohol, and copolymers thereof or cross-linked materials.

Liposomes can be made using any method, e.g., as described in Park, et al., U.S. Pat. Pub. No. 20070042031, including method of producing a liposome by encapsulating a therapeutic product comprising providing an aqueous solution in a first reservoir; providing an organic lipid solution in a second reservoir, wherein one of the aqueous solution and the organic lipid solution includes a therapeutic product; mixing the aqueous solution with said organic lipid solution in a first mixing region to produce a liposome solution, wherein the organic lipid solution mixes with said aqueous solution so as to substantially instantaneously produce a liposome encapsulating the therapeutic product; and immediately thereafter mixing the liposome solution with a buffer solution to produce a diluted liposome solution.

Also provided are nanoparticles comprising compounds used to practice embodiments provided herein (e.g., anti- αvβ3 (avb3) antibodies) to deliver the composition as a drug-containing nanoparticle (e.g., a secondary nanoparticle), as described, e.g., in U.S. Pat. Pub. No. 20070077286. In one embodiment, also provided are nanoparticles comprising a fat-soluble drug provided herein or a fat-solubilized water-soluble drug to act with a bivalent or trivalent metal salt.

### Liposomes

The compositions (e.g., anti- αvβ3 (avb3) antibodies) and formulations used to practice embodiments provided herein can be delivered by the use of liposomes. By using liposomes, particularly where the liposome surface carries ligands specific for target cells, or are otherwise preferentially directed to a specific organ or cell, e.g., cancer stem cells, one can focus the delivery of the active agent into target cells *in vivo.* See, e.g., U.S. Patent Nos. 6,063,400; 6,007,839; Al-Muhammed (1996) J. Microencapsul. 13:293-306; Chonn (1995) Curr. Opin. Biotechnol. 6:698-708; Ostro (1989) Am. J. Hosp. Pharm. 46:1576-1587. For example, in one embodiment, compositions and formulations used to practice embodiments provided herein are delivered by the use of liposomes having rigid lipids having head groups and hydrophobic tails, e.g., as using a polyethyleneglycol-linked lipid having a side chain matching at least a portion the lipid, as described e.g., in US Pat App Pub No. 20080089928. In another embodiment, compositions and formulations used to practice embodiments provided herein are delivered by the use of amphoteric liposomes comprising a mixture of lipids, e.g., a mixture comprising a cationic amphiphile, an anionic amphiphile and/or neutral amphiphiles, as described e.g., in US Pat App Pub No. 20080088046, or 20080031937. In another embodiment, compositions and formulations used to practice embodiments provided herein are delivered by the use of liposomes comprising a polyalkylene glycol moiety bonded through a thioether group and an antibody also bonded through a thioether group to the liposome, as described e.g., in US Pat App Pub No. 20080014255. In another embodiment, compositions and formulations used to practice embodiments provided herein are delivered by the use of liposomes comprising glycerides, glycerol-phospholipids, glycerophosphinolipids, glycerophosphonolipids, sulfolipids, sphingolipids, phospholipids, isoprenolides, steroids, stearines, sterols and/or carbohydrate containing lipids, as described e.g., in US Pat App Pub No. 20070148220.

### Antibodies and Antigen Binding Polypeptides as Pharmaceutical Compositions

Also provided are compositions and methods comprising antibodies or active fragments thereof, or αvβ3-binding polypeptides (e.g., comprising CDRs capable of specifically binding to an αvβ3) capable of specifically binding to an αvβ3 (avb3) integrin polypeptide expressed on a cancer or a tumor cell, or on a CSC, wherein the antibody or polypeptide has an Fc domain or equivalent domain or moiety capable of binding a macrophage and initiating an antibody-dependent cell-mediated cytotoxicity (ADCC) killing of the cell to which the antibody specifically binds (e.g., anti- αvβ3 (avb3) antibodies). In alternative embodiments, provided are compositions to administer these antibodies and polypeptides.

An antibody for practicing embodiments provided herein can comprise a peptide or polypeptide derived from, modeled after or substantially encoded by an immunoglobulin gene or immunoglobulin genes, or fragments thereof, capable of specifically binding an antigen or epitope, see, e.g. Fundamental Immunology, Third Edition, W.E. Paul, ed., Raven Press, N.Y. (1993); Wilson (1994) J. Immunol. Methods 175:267-273; Yarmush (1992) J. Biochem. Biophys. Methods 25:85-97. In alternative aspects, an antibody or polypeptide for practicing embodiments provided herein includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included in the term "antibody."

Method comprise use of monoclonal antibody LM609 (Chemicon Int., Temecula, CA) (CVCL_KS89) (the murine hybridoma having ATCC accession number HB 9537) (see e.g., U.S. patent no. 7,115,261);

Can use "humanized" antibodies, including forms of non-human (e.g., murine) antibodies that are chimeric antibodies comprising minimal sequence (e.g., the antigen binding fragment) derived from non-human immunoglobulin. Humanized antibodies are human immunoglobulins in which residues from a hypervariable region (HVR) of a recipient (e.g., a human antibody sequence) are replaced by residues from a hypervariable region (HVR) of a non-human species (donor antibody) such as mouse, rat, rabbit or nonhuman primate having the desired specificity, affinity, and capacity. Framework region (FR) residues of the human immunoglobulin are replaced by corresponding non-human residues to improve antigen binding affinity.

Humanized antibodies may comprise residues that are not found in the recipient antibody or the donor antibody. These modifications may be made to improve antibody affinity or functional activity. The humanized antibody can comprise substantially all of at least one, and typically two, variable domains, in which all or substantially all of the hypervariable regions correspond to those of a non-human immunoglobulin and all or substantially all of Ab framework regions are those of a human immunoglobulin sequence.

A humanized antibody used to practice embodiments provided herein can comprise at least a portion of an immunoglobulin constant region (Fc), typically that of or derived from a human immunoglobulin.

However completely human antibodies also can be used to practice embodiments provided herein, including human antibodies comprising amino acid sequence which corresponds to that of an antibody produced by a human. This definition of a human antibody specifically excludes a humanized antibody comprising non-human antigen binding residues.

Method comprise use of humanized antibodies capable of specifically binding to an αvβ3 (avb3) integrin polypeptide, including humanized versions of the αvβ3-binding: monoclonal antibody LM609 (Chemicon Int., Temecula, CA) (CVCL_KS89) (the murine hybridoma having ATCC accession number HB 9537) (see e.g., U.S. patent no. 7,115,261

Antibodies used to practice embodiments provided herein comprise "affinity matured" antibodies, e.g., antibodies comprising with one or more alterations in one or more hypervariable regions which result in an improvement in the affinity of the antibody for antigen; e.g., a histone methyl and/or acetyl transferase, compared to a parent antibody which does not possess those alteration(s). Antibodies used to practice embodiments provided herein are matured antibodies having nanomolar or even picomolar affinities for the target antigen, e.g., a histone methyl and/or acetyl transferase. Affinity matured antibodies can be produced by procedures known in the art.

Antibodies used to practice methods as provided herein are: the monoclonal antibody LM609 (Chemicon Int., Temecula, CA) (CVCL_KS89) (the murine hybridoma having ATCC accession number HB 9537) (see e.g., U.S. patent no. 7,115,261); and humanized versions thereof. The monoclonal antibody LM609 is described e.g., in Cheresh et al., J Biol Chem. 1987;262(36):17703-11; and U.S. patent number (USPN) 5,753,230, and USPN 6,590,079. LM609 is a murine monoclonal antibody specific for the integrin αvβ3, see e.g., Cheresh, D. A., Proc. Natl. Acad. Sci. USA 84:6471-6475 (1987), and Cheresh et al, J. Biol. Chem. 262:17703-17711 (1987). LM609 was produced against and is reactive with the M21 cell adhesion receptor now known as the integrin αvβ3. LM609 inhibits the attachment of M21 cells to αvβ3 ligands such as vitronectin, fibrinogen and von Willebrand factor (Cheresh and Spiro, supra) and is also an inhibitor of αvβ3-mediated pathologies such as tumor induced angiogenesis (Brooks et al. Cell 79:1157-1164 (1994)), granulation tissue development in cutaneous wound (Clark et al., Am. J. Pathology, 148:1407-1421 (1996)) and smooth muscle cell migration such as that occurring during restenosis (Choi et al., J. Vascular Surg., 19: 125-134 (1994); Jones et al., Proc. Natl. Acad. Sci. 93:2482-2487 (1996)).

### Kits and Instructions

Also provided are kits comprising compositions (e.g., antibodies) for practicing the methods and uses as provided herein, optionally also including instructions for use thereof. Also provided are kits comprising the monoclonal antibodies or polypeptides capable of specifically binding to an αvβ3, as described herein.

The invention will be further described with reference to the following examples.

### EXAMPLES

### EXAMPLE 1: Immunological targeting of drug resistant lung cancer

This Example and the data presented herein demonstrate the effectiveness of the compositions and methods as provided herein for treating cancers. Provided herein are compositions and methods for treating tumors that have become adapted to the effects of therapy and have become highly aggressive and invasive. This example describes an approach to prevent this process by exploiting two phenomena that arise during acquired drug resistance. First, EGFR mutant lung tumors treated with the RTK inhibitor erlotinib gain expression of integrin αvβ3, and this marker is highly enriched on circulating tumor cells. Second, erlotinib-treated tumors show an accumulation of M2 tumor-associated macrophages, demonstrating how the tumor can manipulate the immune system to support its own proliferation and metastasis. While each of these events drives a more aggressive and drug-resistant tumor phenotype, their coexistence creates a unique opportunity. We demonstrate that systemic delivery of the αvβ3 monoclonal antibody LM609 not only destroys the drug resistant cells within the primary tumor, but eliminates circulating tumor cells. Mechanistically, tumor cell killing occurs by antibody-dependent cell-mediated cytotoxicity (ADCC) for which macrophages are the effector cells *in vitro* and *in vivo.* While erlotinib can initially limit tumor growth, this therapy ultimately creates a more aggressive tumor by enriching for αvβ3/ALDH1A1-expressing cells and recruiting tumor-associated macrophages. By targeting αvβ3, compositions and methods as provided herein, including the exemplary use of LM609, exploits the tumor's manipulation of the immune system to destroy the most drug-resistant cells and halt tumor progression.

Our group previously reported that lung adenocarcinoma patients who had progressed on erlotinib showed a significant increase in expression of integrin αvβ3, that mice bearing αvβ3-negative lung tumors gained expression of αvβ3 as they became resistant to erlotinib (2), and that αvβ3 is highly enriched on metastatic lesions compared to primary tumors from the same patients (3). In fact, we established that integrin αvβ3 is both necessary and sufficient to induce not only erlotinib resistance, but an aggressive stem-like phenotype (2-4). Thus, therapeutically targeting αvβ3 could not only decrease tumor progression but might also eliminate the most drug-resistant cells within the population.

At the molecular level, we identified a role for αvβ3 in this process that was independent from its function as an adhesion receptor (5). As such, strategies to block the canonical integrin function of αvβ3 using cyclic RGD peptides or similar integrin antagonists that compete for integrin-ligand binding do not impact its ability to drive sternness and drug resistance (2). Instead, we reported that disrupting TANK-binding kinase 1 (TBK1)/NF-κB signaling driven by αvβ3 was able to reverse the phenotype of these aggressive tumor cells (2). Because this approach cuts off only one downstream pathway, we considered alternative approaches to directly target and destroy integrin αvβ3-expressing tumor cells.

In addition to function-blocking properties, certain antibodies can mark tumor cells for elimination by antibody-dependent cell-mediated cytotoxicity (ADCC). This process is triggered when the antibody binds to tumor cell antigens and is simultaneously recognized by the FcyR on immune effector cells (6). Considering that other groups have also reported an accumulation of tumor-associated macrophages (TAMs) in erlotinib-resistant tumors (2, 7, 8), provided herein is a strategy to exploit this state of enhanced αvβ3 expression and macrophage enrichment as a tactic to eliminate aggressive αvβ3-expressing tumor cells.

### RESULTS AND DISCUSSION

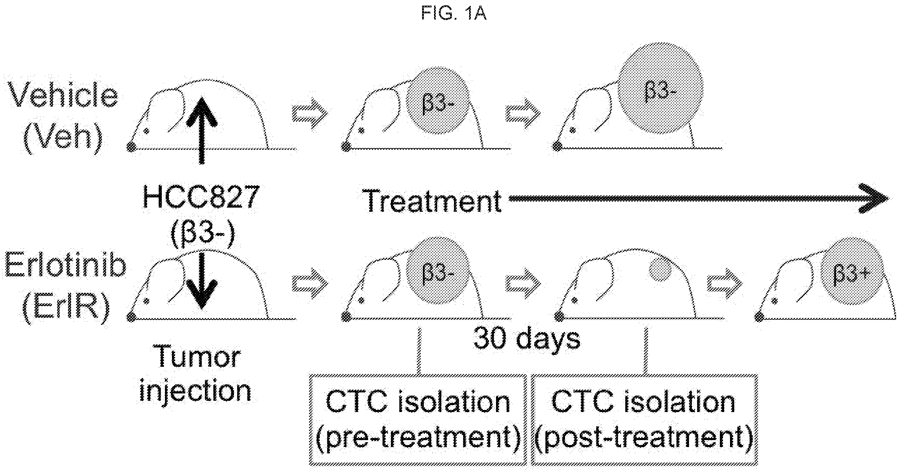

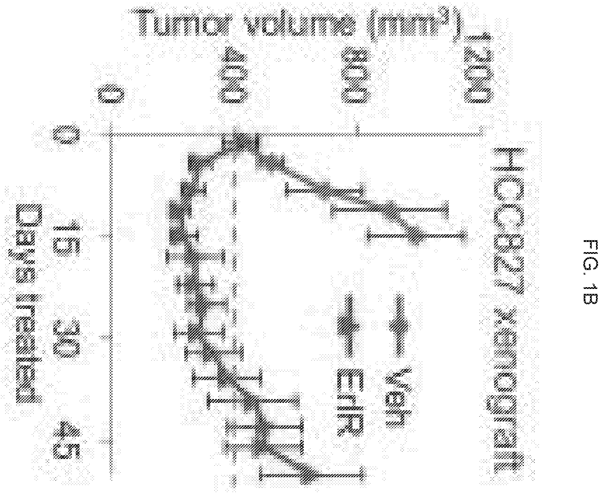

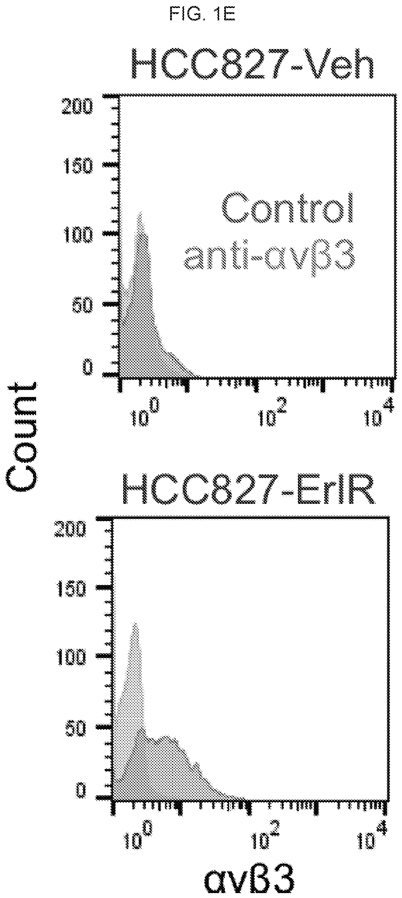

Previous studies in mice and humans reveal that lung cancer resistance to EGFR inhibitors leads to the upregulation of αvβ3 and the acquisition of a drug resistant stem-like fate (2). To evaluate this pathway of acquired resistance, we treated mice bearing αvβ3-negative EGFR-mutant human non-small cell lung cancer (HCC827) subcutaneous xenografts with vehicle or erlotinib, and measured tumor growth over time (Figure 1A). While erlotinib produced the expected tumor growth inhibition within several days, tumors eventually began to re-grow after several weeks (Figure 1B). As previously seen in erlotinib-resistant patients (2), expression of integrin αvβ3 was enriched in the erlotinib-resistant tumors compared to the vehicle group, and the αvβ3-expressing cells were positive for the stem marker aldehyde dehydrogenase 1A1, ALDH1A1 (Figure 1B-D). HCC827-ErlR, a cell line established from an erlotinib-resistant tumor, retained erlotinib resistance *in vitro* and was also resistant to the third-generation EGFR inhibitor, osimertinib (Figure 1E-F). Importantly, we were able to confirm these findings using αvβ3-negative, EGFR-mutant PC9 xenografts.

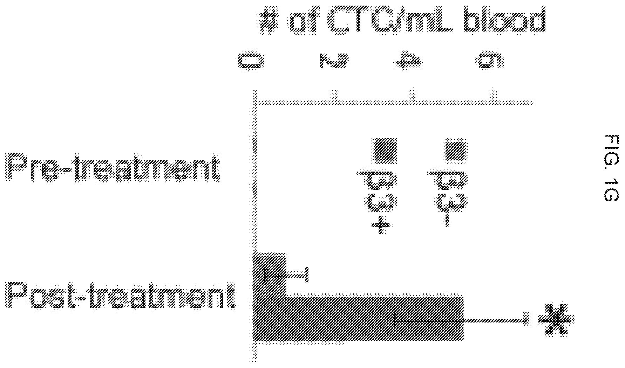

Given that αvβ3 integrin has been linked to tumor progression and metastasis for a range of cancers (3, 9), we reasoned that erlotinib-induced αvβ3 expression may facilitate increased tumor invasion. To evaluate this possibility, we examined the number of circulating tumor cells (CTCs) in HCC827 orthotopic tumor-bearing animals during systemic treatment with erlotinib (Figure 1A). Although tumor growth inhibition in the lung was maintained 30 days after initiating erlotinib treatment, we detected a sharp increase in the number of CTCs in the erlotinib-treated group (Figure 1G-H), suggesting that increased dissemination of tumor cells to the circulation occurs before acquisition of drug resistance in the primary tumor mass. Importantly the vast majority of the CTCs detected following erlotinib treatment were αvβ3 positive (Figure 1G-H). These findings reveal that despite initially controling primary tumor growth, erlotinib has the capacity to convert cells to an αvβ3/ALDH1A1 positive state with the capacity to intravasate and survive in the circulation, highlighting the need to target this population of stem-like, drug-resistant tumor cells that emerges before disease progression is detectable.

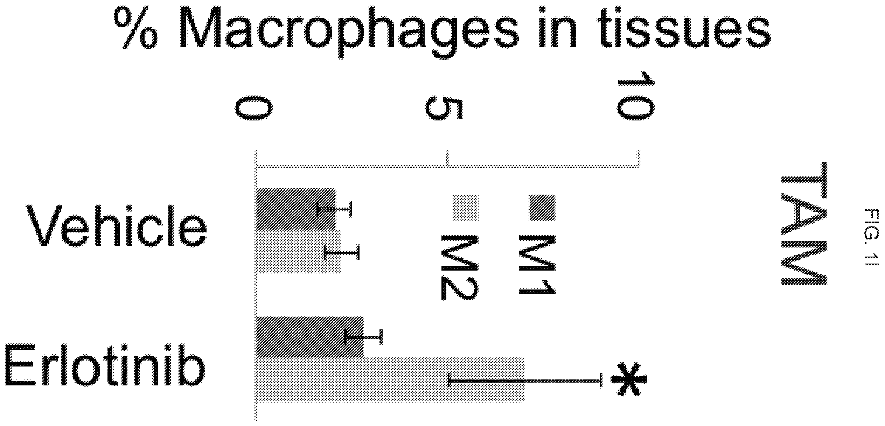

Tumor-associated macrophages (TAMs), particularly the M2-type (10), secrete growth factors, pro-tumorigenic cytokines, and immunosuppressive enzymes to promote tumor progression through induction of angiogenesis, metastasis, and immune suppression (11-14). Accordingly, the observation of enriched TAMs in tumor tissues is associated with poor prognosis in various types of cancer, including lung adenocarcinoma (7). As observed for EGFR inhibitor resistant lung adenocarcinoma patient tumors (7, 8), we found a higher number of TAMs (CD11b-positive, Ly-6G-negative), particularly of the M2 type (CD206-positive or CD206- and MHCll-double negative within the TAM population), within erlotinib-resistant tumors to those from mice treated with a vehicle control (Figure 1I).

The findings above suggest that the erlotinib-resistant tumor environment may be ideally suited for a therapeutic approach that exploits the infiltration of macrophages to target tumor cells. As a strategy to accomplish this, we used an anti-human αvβ3 antibody (LM609) that we previously developed and characterized as an anti-tumor/anti-angiogenic agent in preclinical models (15). In fact, a humanized and affinity-matured form of LM609, etaracizumab, also showed clinical activity in some patients with solid tumors (16-19), although heterogeneity in αvβ3 expression may explain the lack of efficacy in others. Since etaracizumab was reported to mediate antibody-dependent cell mediated cytotoxicity (ADCC) *in vitro* (19), we considered LM609 as a unique opportunity to engage TAMs to target the enriched population of tumor cells that emerge during acquired resistance to erlotinib *in vivo.*

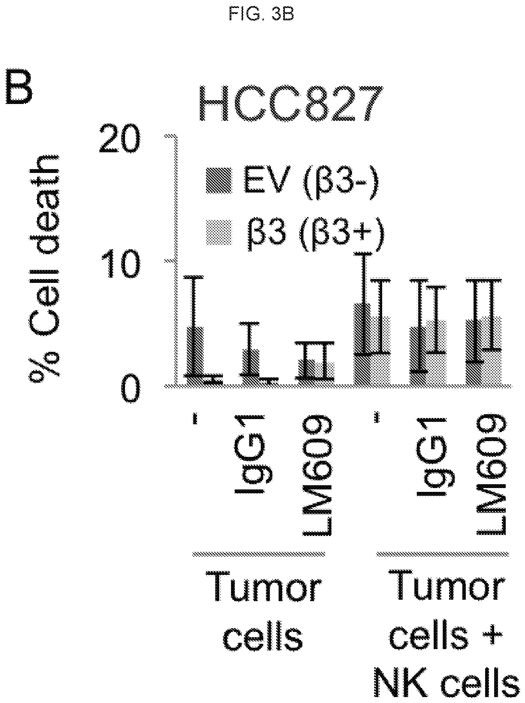

While erlotinib alone enriched for αvβ3 and led to tumor regrowth and the appearance of CTCs, the combination of erlotinib and LM609 maintained erlotinib sensitivity (Figure 2A). Not only did LM609 eliminate αvβ3-positive tumor cells (Figure 2B), but it also prevented the increase in αvβ3-positive CTCs (Figure 2C-E), suggesting that LM609 prevents both erlotinib resistance and the intravasation/ survival of circulating αvβ3-positive tumor cells during the course of therapy. While integrin αvβ3 functions as a cell-matrix adhesion molecule (15), treatment with LM609 alone did not affect viability of either αvβ3-positive or -negative cells. This finding is consistent with other studies that showed the role of αvβ3 integrin in tumor cells being independent from its function as an adhesion molecule and rather induction of anchorage independent growth (2, 3). Instead, we found that LM609 increased αvβ3-positive cell death by ADCC, and we determined that this process was mediated by macrophages, but not NK cells (Figure 3A and 3B).

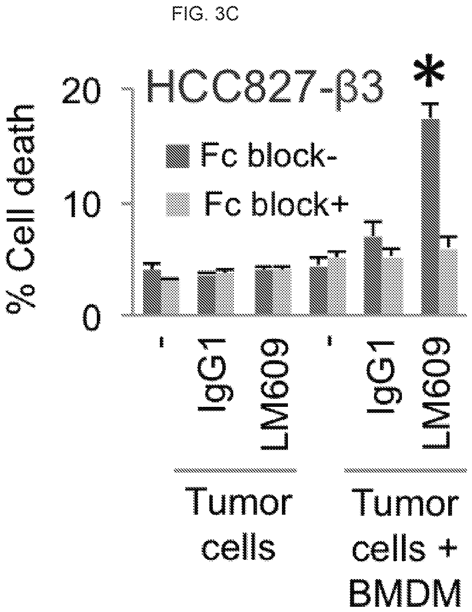

Indeed, blockade of Fcγ receptors on macrophages diminished the effect of LM609 *in vitro* (Figure 3C). While LM609 suppressed growth of αvβ3-positive tumors *in vivo,* depletion of macrophages using clodronate liposomes abolished this effect (Figure 3D). Together, these studies point to macrophage-mediated ADCC as a primary mechanism of activity for LM609 both *in vitro* and *in vivo.*

Our study demonstrates how erlotinib therapy changes the tumor by enriching for αvβ3 expression, and highlights how the tumor can manipulate the host immune system to support a drug-resistant phenotype. While these adaptations may facilitate tumor progression during treatment with erlotinib, they also provide a unique opportunity to exploit this scenario. Indeed, an anti-αvβ3 antibody encourages macrophages to selectively destroy the drug-resistant αvβ3/ALDH1A1-expressing tumor cells within the primary tumor as well as CTCs, together delaying the onset of acquired resistance (Figure 2A). Because LM609 only recognizes human integrin αvβ3, our xenograft model allows us to separate its role in ADCC from any impact on tumor-associated endothelial cells that require this integrin for angiogenesis (20, 21). Considering that angiogenesis contributes to tumor growth, and since LM609 and etaracizumab are IgG1 antibodies with higher affinity for human versus mouse macrophage Fcγ receptors (22), our preclinical evaluation may underestimate the efficacy of this therapeutic strategy for erlotinib-resistant tumors in humans. Furthermore, given that not only erlotinib but also other cancer therapeutics increase β3 integrin expression in epithelial cancer cells (Figure 6), anti-αvβ3 antibody therapy can be used with various therapeutics. In conclusion, provided herein are combinations anti-αvβ3 antibody with EGFR inhibitors and/or other cancer drugs as a unique therapeutic approach for epithelial cancer patients who progress on a standard of therapy.

### METHODS

### Cells lines and reagents

Lung adenocarcinoma (HCC827 in RPMI) cells were obtained from ATCC. The melanoma cell line, M21, was a gift from Dr. D. L. Morton (University of California, Los Angeles, USA). The lung adenocarcinoma cell line, PC9, was a gift from Dr. Joan Massague (Sloan-Kettering Institute, USA). Cell line authentication was performed by the ATCC using short tandem repeat DNA profiles. Upon receipt, each cell line was expanded, cryopreserved as low-passage stocks, and tested routinely for *Mycoplasma.* For ectopic expression and genetic knockdown, cells were transfected with vector control (GFP labeled), integrin β3, or luciferase using a lentiviral system as previously described (1). Levels of αvβ3 integrin were tested by flow cytometry as described below.

Captisol was obtained from Cydex^{™} (NC0604701) and diluted in water at 6 %. Erlotinib was obtained from SellekChem^{™} (S1023) and diluted in DMSO for *in vitro* or in captisol for *in vivo* experiments. LM609 was produced in this laboratory as previously described (2). The activity was confirmed by adhesion assay as described below. Control and clodronate liposome solutions were obtained from ClodronateLiposome.com.

### Adhesion assay

M21 (αvβ3+) cells were plated on a vitronectin-coated or collagen-coated plate with and without LM609. Adherent cells were stained with crystal violet and the visible absorbance (600 nm) of each well was quantified using a microplate reader.

### Erlotinib resistant lung adenocarcinoma xenograft model

Erlotinib resistant lung adenocarcinoma xenograft model was utilized as previously described (3). Briefly, HCC827 (5 × 10⁶ tumor cells in 100 µl of RPMI) cells were subcutaneously injected to the right flank of female nu/nu mice (8 - 10 weeks old). Tumors were measured twice per week with calipers (tumor volume (mm³) = length × width × width/2). Animals with a tumor volume of 250 - 700 mm³ were randomly assigned into groups treated with captisol (oral, six times/week) and PBS (i.p., twice/week), captisol and LM609 (i.p., 10 mg/kg, twice/week), erlotinib (oral, 6.25 mg/kg, six times/week) and PBS, or erlotinib and LM609. The mice in the captisol groups were sacrificed on day 15 due to the large tumor size. The erlotinib groups were sacrificed on day 50. Tumor tissues were divided into three pieces for snap freezing, freezing in OCT compound (VWR, 25608-930), or 10 % formalin fixation (Fisher Scientific, 23-313095) for further analyses.

### Quantification of TAMs

TAMs were isolated from tumor tissues as previously described (4). Snap frozen tumor tissues were thawed, minced, and dissociated in HBSS containing collagenase IV (Sigma, C5138, 0.5 mg/mL), hyaluronidase (Sigma, H2654, 0.1 mg/mL), dispase ll (Roche, 04942078001, 0.6 U/mL), and DNase IV (Millipore, 260913- 10MU, 5 U/mL) at 37°C for 15 minutes. Cell suspensions were filtered through 70 µm cell strainer and washed with PBS. Single cell suspensions (10⁶ cells/100 µL in 5 % BSA in PBS) were incubated with Mouse BD Fc Block^{™} (BD Biosciences, 553142, 1:50) for 10 minutes at 4°C and fluorescently labeled antibodies, CD11b (eBioscience, 17-0112-81, 1:100), Ly-6G(eBioscience, 25-5931, 1:100), CD206 (BioRad, MCA2235PET), and MHCll (BD Biosciences, 562928) for one hour at 4°C. Flow cytometry was performed on BD LSRFortessa^{™}, and the ratio of TAMs (CD11b-positive, Ly-6G-negative), M1 (CD206-negative, MHCll-positive within TAMs), and M2 (non-M1 population within TAMs) in the tumor tissue was calculated using the flow cytometry analysis program FlowJo^{™} (Treestar).

### Immunohistochemical analysis

Immunohistochemical staining was performed on unstained FFPE slides according to the manufacturers recommendations for the VECTASTAIN Elite ABC HRP Kit (Vector Laboratories, PK-6100). An anti-integrin β3 antibody (Cell Signaling, 13166, 1:200) and a biotinylated goat anti-rabbit antibody (Vector Laboratories, BA- 1000, 1:200) were used. The stained tissues were imaged on NanoZoomer Slide Scanning System^{™} (Hamamatsu), and the β3-positive area fraction with respect to tumor tissue was calculated utilizing ImageJ (NIH) (5).

### Orthotopic lung adenocarcinoma model and CTC isolation

Luciferase- and GFP-positive HCC827 cells (5 × 10⁶ cells in 50 µl of PBS) were injected into the right lungs of female nu/nu mice (8 - 10 weeks old). The tumor growth was monitored by IVIS SPECTRUM^{™} (PerkinElmer) every four weeks. Eight weeks after the injection, the mice were randomly divided into three groups, pre-treatment, erlotinib (oral, 6.25 mg/kg, six times/week) treatment, and the combination of erlotinib and LM609 (i.p., 10 mg/kg, twice/week). The pre-treatment group mice were sacrificed in week 8 for CTC analysis. The treatment group mice were treated with erlotinib and or LM609 for four weeks before being sacrificed. Whole blood (0.5 mL/mouse) was collected in EDTA tubes, and PBMCs including CTCs were isolated using Lymphoprep^{™} (STEMCELL Technologies, 85415) and SepMate^{™} (STEMCELL Technologies, 07801) following the manufacturer's protocol. Isolated cells were washed with PBS, plated on poly-L-Lysine (Sigma, P1399) coated 8-well chamber plates, fixed with 4 % PFA (VWR, AA43368-9M), and stained with DAPI (Life Technologies, D1306, 1 µg/mL in 1 % BSA in PBS), LM609 (5 µg/mL in 1 % BSA in PBS), and a fluorescently-labeled secondary antibody (Thermo, A21235, 1:500). The cells were analyzed utilizing Nikon Eclipse C2^{™} confocal microscope (Nikon). CTCs were defined as DAPI and GFP double positive cells. The numbers of αvβ3-positive and -negative CTCs per mouse were counted. After sacrificing the mice, the tumor mass volume in the lungs were also measured utilizing an OV100^{™} fluorescence imaging system (Olympus).

### Establishment of erlotinib induced αvβ3-positive cell lines

Erlotinib resistant HCC827 (HCC827-R) cells and PC9 (PC9-R) cells were established as previously described (3). Resistance to EGFR inhibitors (erlotinib and AZD9291) was confirmed by MTT assays (Figure 1B).

### MTT assay

Cells were plated in 96-well plates, and after appropriate treatments, the cells were incubated in MTT solution (Sigma, M2128, 0.5 mg/mL in growth media) for two hours at 37°C. Then, the MTT solution was removed, and the blue crystalline precipitate in each well was dissolved in DMSO. Visible absorbance of each well at 560 nm was quantified using a microplate reader.

### Bone marrow derived macrophage (BMDM) isolation

BMDMs were aseptically harvested from 8-10 week-old female C57BL/6 mice by flushing leg bones of euthanized mice with RPMI, filtering through 70 µm cell strainers, and incubating in Red Blood Cell Lysing Buffer Hybri-Max^{™} (Sigma, R7757).

### NK cell isolation

Splenocytes were aseptically harvested from 8-10 week-old female C57BL/6 mice by mincing spleens of euthanized mice with PBS containing 2 % FBS and 1 mM EDTA, filtering through 40 µm strainers, and incubating in Red Blood Cell Lysing Buffer Hybri-Max^{™} (Sigma, R7757). The NK cells were isolated from the splenocytes using NK Cell Isolation Kit II^{™} (Miltenyi, 130-096-892).

### ADCC assay

Target cells stained with CFSE Cell Division Tracker Kit^{™} (BioLegend, 423801) or CellTrace^{™} Far Red Cell Proliferation Kit^{™} (ThermoFisher, C34564) were co-cultured with BMDMs or NK cells with or without isotype IgG or LM609 for five hours at 37°C. After the incubation, the cells were stained with PI (Sigma, P4864, 1:1000), and flow cytometry was performed on BD LSRFortessa^{™}. The ratio of dead target cells (PI-positive) to the total target cell population (CFSE- or far red-positive) was calculated as previously described (6).

### Lung adenocarcinoma xenograft model with macrophage depletion

HCC827-β3 (5 × 10⁶ tumor cells in 100 µl of RPMI) cells were subcutaneously injected to the right flank of female nu/nu mice (8 - 10 weeks old). Tumors were measured twice per week with calipers. Animals with a tumor volume of 30 - 110 mm³ were randomly assigned into groups treated with control liposome (i.p., 200 µL, twice/week) and PBS (i.p., twice/week, n = 5), control liposome (i.p., 200 µL, twice/week) and LM609 (i.p., 10 mg/kg, twice/week, n = 6), clodronate liposome (i.p., 200 µL, twice/week) and PBS (i.p., twice/week, n = 5), or clodronate liposome (i.p., 200 µL, twice/week) and LM609 (i.p., 10 mg/kg, twice/week, n = 6). After 15 days of the treatments, the tumor tissues were fixed and frozen in OTC compound.

### Immunofluorescence

Immunofluorescence staining was performed on unstained OCT slides. The slides were permeabilized with 0.1 % TritonX-100 (Bio-Rad, 1610407) in PBS for one minute, blocked with 10 % NGS (Jackson ImmunoResearch, 005-000-121) in PBS for two hours, and incubated with DAPI (Life Technologies, D1306, 1 µg/mL in 1 % BSA in PBS) and an anti-mouse F4/80 antibody (eBioscience, 14-4801, conjugated with TexasRed fluorocore by OneWorldLab) for two hours at room temperature. Confocal microscopy pictures were taken utilizing Nikon Eclipse C2^{™} confocal microscope (Nikon). F4/80-positive area fraction with respect to tumor tissue was calculated utilizing ImageJ^{™} (NIH) (5).

### Flow cytometry for αvβ3 integrin

Cell pellets were washed blocked with 1 % BSA in PBS for 30 minutes at room temperature and stained with LM609 (5 µg/mL in 1 % BSA in PBS) and a fluorescently labeled secondary antibody (Thermo, A21235, 1:500). After the staining, the cells were incubated with PI (Sigma, P4864, 1: 1000), and flow cytometry was performed on BD LSRFortessa^{™}. The levels of αvβ3 integrin were analyzed using the flow cytometry analysis program FlowJo^{™} (Treestar).

### Gene expression

Total RNA was collected using the RNeasy^{™} RNA Purification kit (Qiagen). cDNA was synthesized with MultiScribe^{™} reverse transcriptase using random primers (Thermo), and PCR with reverse transcription was carried out on a LightCycler^{™} with SYBR Green probes (Roche). Expression relative to reference genes was calculated using the 2-ddCT method.

### Study approval

All research was conducted under protocol S05018 and approved by the UCSD Institutional Animal Care and Use Committee. All studies are in accordance with the guidelines set forth in the NIH Guide for the Care and Use of Laboratory Animals.

### Statistical analysis

Student's *t* test, Mann-Whitney U test, or ANOVA was performed to compare independent sample groups. Kaplan-Meier estimator was performed to measure resistance-free survival. Excel (Microsoft) and SPSS (IBM Analytics) were utilized for statistical analysis.

### Figure Legends

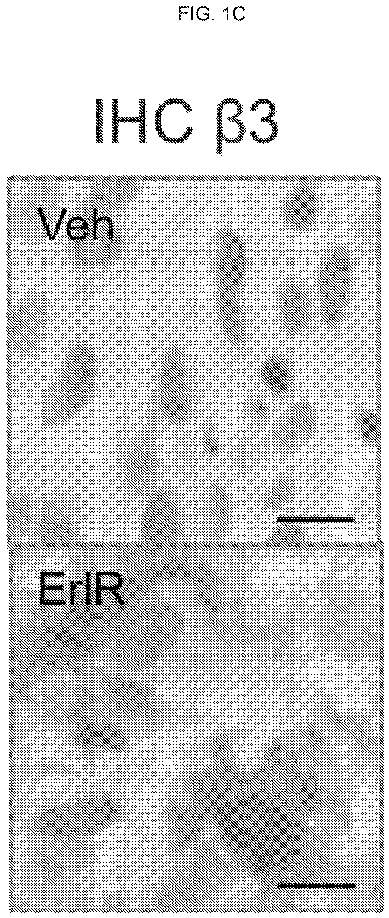

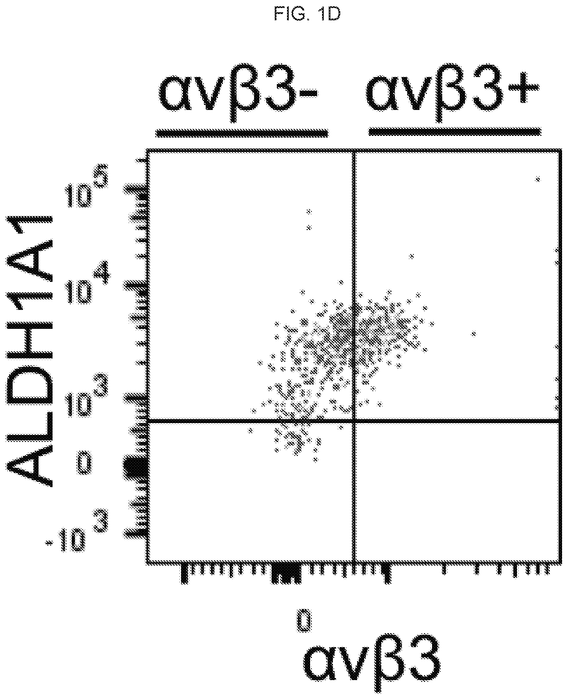

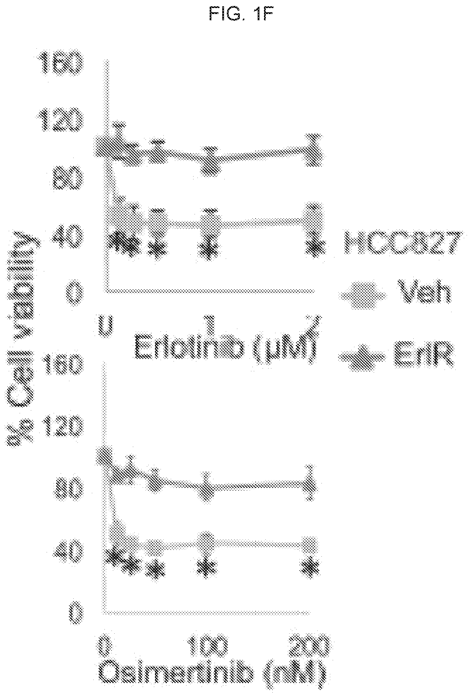

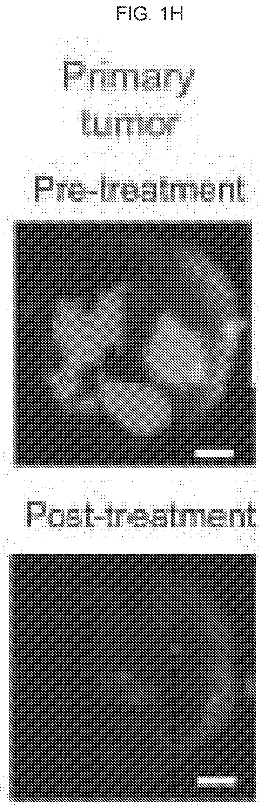

### Figure 1. Erlotinib resistance impacted the primary tumor, CTC, and stroma.

FIG. 1A: A schematic of the erlotinib resistant xenograft model.

FIG. 1B-D: Erlotinib resistant tissues display increased αvβ3/ALDH1A1-expressing cells. HCC827 (β3-) xenograft mice were treated with the vehicle (Veh, n=5), or erlotinib (ErlR, n=9). The tumor growth was measured (FIG. 1B). The tumor tissues were stained for β3 (FIG. 1C). Bars, 10 µm. Cells isolated from erlotinib resistant tissues were stained for αvβ3 and ALDH1A1 for flow cytometry (FIG. 1D). The graph represents two mice.

FIG. 1E-F: Erlotinib resistant αvβ3-positive cells were resistant to a third generation EGFR inhibitor, osimertinib. Tumor cells isolated from a vehicle treated mouse (veh) and an erlotinib treated mouse (ErlR) were stained for αvβ3 for flow cytometry (FIG. 1E). The graphs are reperesentatives of three experiments. The cells were treated with erlotinib or osimertinib for 72 hours, and cell viability was measured (FIG. 1F).

FIG. 1G-H: Erlotinib increased CTCs while the primary tumors are still responding to the treatment. Eight weeks after the lung injection of HCC827 (β3-, GFP+, luciferase+) cells, the mice were treated with erlotinib for 30 days before CTCs isolation. The CTCs were stained for αvβ3. αvβ3-positive (β3+) and -negative (β3-) CTCs from mice before (pre-treatment, n=3) and after the treatment (post-treatment, n=5) were counted (FIG. 1G). The primary masses were visualized (FIG. 1H). GFP, tumor tissue; Bars, 5 mm.

FIG. 1I: graphically illustrates data showing how M2-TAMs were increased in the elrotinib resistant xenograft tissues; percentage of M1 (dark grey), and M2-TAMs (light grey)) in the tumor tissues was compared between the vehicle (n=9) and erlotinib (n=9) treatments after 50 days of the treatments. Two-tailed Student's t test was used to determine statistical significance (*P<0.05 compared to controls). Error bars indicate standard errors.

### Figure 2. LM609 produced a less aggressive phenotype.

FIG. 2A: LM609 inhibited acquisition of erlotinib resistance. HCC827 (β3-) xenograft mice were treated with erlotinib (Erlotinib, n=9) or erlotinib and LM609 (Erlotinib+LM609, n=10). The tumor growth was measured twice weekly. Progression free survival was analyzed utilizing Kaplan Meier estimator.

FIG. 2B: LM609 eliminated β3-positive cells. HCC827 (β3-) xenograft mice were treated with Captisol and PBS (control, n=5), Captisol and LM609 (LM609, n=5), erlotinib and PBS (Erlotinib, n=9), or erlotinib and LM609 (Erl+LM609, n=10) for 50 days. Tumor tissues were stained for integrin β3, and β3-positive area in the tissues were quantified (n=9 fields per group) (upper panel). Pictures (lower panel) are representatives. Bars, 10 µm. Two-tailed Student's t test was used to determine statistical significance (*P<0.05). Error bars indicate standard deviations.

FIG. 2C-E: LM609 decreased numbers of CTCs induced by erlotinib. Eight weeks after the right lung injection of HCC827 (β3-, GFP+, luciferase+) cells, the mice were treated with erlotinib or the combination of erlotinib and LM609 (erlotinib+LM609) for four weeks before CTCs were isolated. The tumor volume was measured by bioluminescence using IVIS Spectrum before the treatments (Pre-treatment, n=14) and after the treatment with erlotinib (Post-erlotinib, n=4) or the combination of erlotinib and LM609 (Post-Erl+LM609, n=5) and quantified (FIG. 2C). The pictures are representatives (FIG. 2D). The CTCs were stained for αvβ3 to quantify αvβ3-positive (β3+) and -negative (β3-) CTCs (FIG. 2E). An example of αvβ3-positive CTCs is shown (lower right panel). Two-tailed Student's *t* test was used to determine statistical significance (*P<0.05 compared to the pre-treatment). Error bars indicate standard errors (upper panel) and standard deviations (lower panel). Bars, 5 mm (upper panel); bars, 10 µm (lower panel); blue, DAPI; green, GFP; red, αvβ3.

### Figure 3. LM609 eliminated αvβ3-positive cells via macrophage-mediated ADCC.

FIG. 3A-C: LM609 eliminated αvβ3-positive cells *via* macrophage-mediated ADCC *in vitro.* ADCC assays with bone marrow derived macrophages (BMDMs) or NK cells were performed with cancer cells with and without αvβ3 integrin treated with the IgG isotype or LM609 10 µg/mL and/or Fc blocker. Percent cell death of cancer cells was quantified utilizing propidium iodide by flow cytometry. Effector and target cell ratio was 5:1. Two-tailed Student's *t* test was used to determine statistical significance (*P<0.05 compared to IgG isotype controls). Error bars indicate standard errors. Empty vector, EV; β3 plasmid, β3; Veh, cells isolated from a vehicle treated tissue; ErlR, cells isolated from an erlotinib resistant tissue.

FIG. 3D-E: LM609 eliminated αvβ3-positive tumor growth only in the mice that have macrophages. Nude mice were subcutaneously injected with β3 ectopically expressing HCC827 cells and treated with control liposome and PBS (FIG. 3D left panel, control, n=5), control liposome and LM609 (FIG. 3D left panel, LM609, n=6), clodronate liposome and PBS (right panel, control, n=5), or clodronate liposome and LM609 (FIG. 3D right panel, LM609, n=6). Tumor growth was monitored for 15 days, and after the treatments, F4/80 staining in the tumor tissues was quantified (FIG. 3D right panel, n=10 fields per group). Two-tailed Student's *t* test was used to determine statistical significance (*P<0.05 compared to the control). Error bards indicate standard errors.

### Figure 4: Erlotinib resistant tumor cells gained αvβ3 integrin and were resistant to osimertinib.

FIG. 4A: Erlotinib treated tumor gained resistance. PC9 (β3-) subcutaneous xenograft mice were treated with the vehicle (Veh) (n=1) or erlotinib (ErlR, n=1). The tumor growth was measured weekly.

FIG. 4B: The cells from erlotinib resistant tissue αvβ3-positive while the cells from the vehicle treated animal were not. Levels of αvβ3 on cells isolated from the vehicle treated (Veh) and erlotinib treated (ErlR) animals were measured by flow cytometry. Blue, secondary control; red, stained with LM609.

FIG. 4C: Erlotinib resistant cells were osimertinib resistant. Cells from the vehicle treated (Veh) and erlotinib treated (ErlR) animals were treated with erlotinib or osimertinib at the indicated doses and MTT assay was performed to measure cell viability.

Two-tailed Student's *t* test was used to determine statistical significance (*P<0.05 compared to the control). Error bars indicate standard deviations.

### Figure 5: LM609 inhibited ligand binding of αvβ3 integrin but not cell viability.

FIG 5A: LM609 inhibited ligand binding of integrin αvβ3. M21 cells (αvβ3+) were plated on collagen or vitronectin (αvβ3 ligand) with indicated doses of LM609. The adherent cell number was measured.

FIG. 5B: LM609 did not affect cell viability. Paired αvβ3-positive (β3+) and -negative (β3-) cells were treated with indicated doses of LM609, and MTT assay was performed to measure cell viability.

Two-tailed Student's *t* test was used to determine statistical significance. Error bars indicate standard deviations. EV, empty vector; β3, β3 plasmid; Veh, cells isolated from the vehicle group (erlotinib sensitive); ErlR, cells isolated from the erlotinib resistant tissue.

FIG. 6 graphically illustrates data showing that cancer therapeutics enrich β3 integrin in epithelial cancer cells: qPCR was performed to detect mRNA expression of integrin α and β subunits in response to increasing dose of hydrogen peroxide (H2O2) for 72h, expressed as fold relative to vehicle control. Error bars indicate standard deviations. *P<0.05 compared to controls.

### References

1. Holohan et al., Cancer drug resistance: an evolving paradigm. Nat Rev Cancer. 2013;13(10):714-26.
2. Seguin et al., An integrin beta(3)-KRAS-RalB complex drives tumour sternness and resistance to EGFR inhibition. Nat Cell Biol. 2014;16(5):457-68.
3. Desgrosellier et al., An integrin alpha(v)beta(3)-c-Src oncogenic unit promotes anchorage-independence and tumor progression. Nat Med. 2009;15(10):1163-9.
4. Desgrosellier et al., Integrin alphavbeta3 drives slug activation and sternness in the pregnant and neoplastic mammary gland. Dev Cell. 2014;30(3):295-308.
5. Smith JW, and Cheresh DA. Integrin (alpha v beta 3)-ligand interaction. Identification of a heterodimeric RGD binding site on the vitronectin receptor. J Biol Chem. 1990;265(4):2168-72.
6. Carter P. Improving the efficacy of antibody-based cancer therapies. Nat Rev Cancer. 2001;1(2):118-29.
7. Chung et al., Tumor-associated macrophages correlate with response to epidermal growth factor receptor-tyrosine kinase inhibitors in advanced non-small cell lung cancer. Int J Cancer. 2012;131(3):E227-35.
8. Zhang et al., M2-polarized macrophages contribute to the decreased sensitivity of EGFR-TKIs treatment in patients with advanced lung adenocarcinoma. Med Oncol. 2014;31(8):127.
9. Knowles et al.,Integrin alphavbeta3 and fibronectin upregulate Slug in cancer cells to promote clot invasion and metastasis. Cancer Res. 2013;73(20):6175-84.
10. Biswas SK, and Mantovani A. Macrophage plasticity and interaction with lymphocyte subsets: cancer as a paradigm. Nat Immunol. 2010; 11 (1 0): 889-96.
11. Riabov et al., Role of tumor associated macrophages in tumor angiogenesis and lymphangiogenesis. Front Physiol. 2014;5(75.
12. Trikha et al., E2f3 in tumor macrophages promotes lung metastasis. Oncogene. 2016;35(28):3636-46.
13. Noy R, and Pollard JW. Tumor-associated macrophages: from mechanisms to therapy. Immunity. 2014;41(1):49-61.
14. Kaneda et al., PI3Kgamma is a molecular switch that controls immune suppression. Nature. 2016;539(7629):437-42.
15. Cheresh DA. Human endothelial cells synthesize and express an Arg-Gly-Asp-directed adhesion receptor involved in attachment to fibrinogen and von Willebrand factor. Proc Natl Acad Sci USA. 1987;84(18):6471-5.
16. Delbaldo et al.,. Phase I and pharmacokinetic study of etaracizumab (Abegrin), a humanized monoclonal antibody against alphavbeta3 integrin receptor, in patients with advanced solid tumors. Invest New Drugs. 2008;26(1):35-43.
17. McNeel et al., Phase I trial of a monoclonal antibody specific for alphavbeta3 integrin (MEDI-522) in patients with advanced malignancies, including an assessment of effect on tumor perfusion. Clin Cancer Res. 2005;11(21):7851-60.
18. Hersey et al., A randomized phase 2 study of etaracizumab, a monoclonal antibody against integrin alpha(v)beta(3), + or - dacarbazine in patients with stage IV metastatic melanoma. Cancer. 2010;116(6):1526-34.
19. Mulgrew et al.,Direct targeting of alphavbeta3 integrin on tumor cells with a monoclonal antibody, Abegrin. Mol Cancer Ther. 2006;5(12):3122-9.
20. Brooks et al., Requirement of vascular integrin alpha v beta 3 for angiogenesis. Science. 1994;264(5158):569-71.
21. Brooks et al., Integrin alpha v beta 3 antagonists promote tumor regression by inducing apoptosis of angiogenic blood vessels. Cell. 1994;79(7):1157-64.
22. Guilliams et al., The function of Fcgamma receptors in dendritic cells and macrophages. Nat Rev Immunol. 2014;14(2):94-108.

### Supplemental References

1. Seguin L, Kato S, Franovic A, Camargo MF, Lesperance J, Elliott KC, Yebra M, Mielgo A, Lowy AM, Husain H, et al. An integrin beta3-KRAS-RalB complex drives tumour sternness and resistance to EGFR inhibition. Nat Cell Biol. 2014;16(5):457-68.
2. Cheresh DA, and Spiro RC. Biosynthetic and functional properties of an Arg-Gly-Asp-directed receptor involved in human melanoma cell attachment to vitronectin, fibrinogen, and von Willebrand factor. J Biol Chem. 1987;262(36):17703-11.
3. Seguin L, Kato S, Franovic A, Camargo MF, Lesperance J, Elliott KC, Yebra M, Mielgo A, Lowy AM, Husain H, et al. An integrin beta(3)-KRAS-RalB complex drives tumour sternness and resistance to EGFR inhibition. Nat Cell Biol. 2014;16(5):457-68.
4. Kaneda MM, Messer KS, Ralainirina N, Li H, Leem CJ, Gorjestani S, Woo G, Nguyen AV, Figueiredo CC, Foubert P, et al. PI3Kgamma is a molecular switch that controls immune suppression. Nature. 2016;539(7629):437-42.
5. Shu JQ, G.; Mohammad, I. A Semi-Automatic Image Analysis Tool for Biomarker Detection in Immunohistochemistry Analysis. Seventh International Conference on Image and Graphics. 2013:937- 42.
6. Bracher M, Gould HJ, Sutton BJ, Dombrowicz D, and Karagiannis SN. Three-colour flow cytometric method to measure antibody-dependent tumour cell killing by cytotoxicity and phagocytosis. J Immunol Methods. 2007;323(2):160-71.

## Claims

1. A pharmaceutical composition or a formulation for use for
killing or reducing the number of αvβ3 (avb3) polypeptide-expressing drug resistant cancer cells or tumor cells or drug resistant Cancer Stem Cells (CSCs) in an individual in need thereof,
wherein the pharmaceutical composition or a formulation comprises:
an integrin αvβ3 antibody capable of specifically binding to an αvβ3 (avb3) integrin polypeptide expressed on the drug resistant cancer or the drug resistant tumor cell, or on the drug resistant CSC, wherein the antibody has an Fc domain or equivalent domain or moiety capable of binding a macrophage and initiating an antibody-dependent cell-mediated cytotoxicity (ADCC) killing of the cell to which the antibody specifically binds, wherein the antibody is the monoclonal antibody LM609, or a humanized version thereof, and
wherein the αvβ3 (avb3) polypeptide-expressing drug resistant cancer cells, tumor cells or Cancer Stem Cells (CSCs) are resistant to a Receptor Tyrosine Kinase (RTK) inhibitor.

2. The composition for the use of claim 1, wherein the macrophage is a human macrophage, or a tumor associated macrophage (TAM).

3. The composition for the use of any of the preceding claims, wherein the cancer is an epithelial cancer or epithelial tumor cell.

4. The composition for the use of any of the preceding claims, wherein the antibody is
administered intravenously, intramuscularly or subcutaneously to the individual in need thereof.

5. The composition for the use of any of the preceding claims, wherein the antibody is formulated as a sterile pharmaceutical composition or formulation, or is formulated for administration intravenously, intramuscularly or subcutaneously.

6. The composition for the use of any of the preceding claims, wherein the dosage of antibody is based on either fixed or body weight-based dosing, or a fixed dose of between about 100 and 1200 mg monthly, or at a dosage of between about 0.3 to 10 mg/kg.

7. The composition for the use of any of the preceding claims, wherein a growth factor inhibitor is also administered, wherein optionally the growth factor inhibitor comprises a Receptor Tyrosine Kinase (RTK) inhibitor, a Src inhibitor, an anti-metabolite inhibitor, a gemcitabine, a GEMZAR^{™}, a mitotic poison, a paclitaxel, a taxol, an ABRAXANE^{™}, an erlotinib, a TARCEVA^{™}, a lapatinib, a TYKERB^{™}, a cetuxamib, an ERBITUX^{™}, or an insulin growth factor inhibitor.

8. A kit comprising the pharmaceutical composition or the formulation for the use of anyone of claims 1-7.

## Patentansprüche

1. Pharmazeutische Zusammensetzung oder Rezeptur zur Verwendung zum Abtöten oder Verringern der Anzahl von αvβ3 (avb3) Polypeptidexprimierenden arzneimittelresistenten Krebszellen oder Tumorzellen oder arzneimittelresistenten Krebsstammzellen (CSCs) bei einer Person, die dies benötigt,
wobei die pharmazeutische Zusammensetzung oder Rezeptur umfasst:
einen Integrin- αvβ3-Antikörper, der zum spezifischen Binden an ein αvβ3 (avb3)-Integrin-Polypeptid imstande ist, das auf dem arzneimittelresistenten Krebs oder der arzneimittelresistenten Tumorzelle oder auf dem arzneimittelresistenten CSC exprimiert wird, wobei der Antikörper eine Fc-Domäne oder eine äquivalente Domäne oder einen Anteil aufweist, der zum Binden eines Makrophagen und zum Initiieren einer antikörperabhängigen zellvermittelten Zytotoxizität (ADCC) imstande ist, welche die Zelle, an die der Antikörper spezifisch bindet, abtötet, wobei der Antikörper der monoklonale Antikörper LM609 oder eine humanisierte Version davon ist, und
wobei die αvβ3 (avb3)-Polypeptid-exprimierenden arzneimittelresistenten Krebszellen, Tumorzellen oder Krebsstammzellen (CSCs) gegen einen Rezeptortyrosinkinase (RTK)-Inhibitor resistent sind.

2. Zusammensetzung zur Verwendung nach Anspruch 1, wobei der Makrophage ein humaner Makrophage oder ein tumorassoziierter Makrophage (TAM) ist.

3. Zusammensetzung zur Verwendung nach einem der vorhergehenden Ansprüche, wobei der Krebs ein Epithelkarzinom oder eine Epitheltumorzelle ist.

4. Zusammensetzung zur Verwendung eines der vorhergehenden Ansprüche, wobei der Antikörper der Person, die diesen benötigt, intravenös, intramuskulär oder subkutan verabreicht wird.

5. Zusammensetzung zur Verwendung nach einem der vorhergehenden Ansprüche, wobei der Antikörper als sterile pharmazeutische Zusammensetzung oder Rezeptur formuliert ist oder zur intravenösen, intramuskulären oder subkutanen Verabreichung formuliert ist.

6. Zusammensetzung zur Verwendung nach einem der vorhergehenden Ansprüche, wobei die Dosierung des Antikörpers entweder auf einer festen oder körpergewichtsbezogenen Dosierung oder einer festen Dosis zwischen etwa 100 und 1200 mg monatlich oder einer Dosierung zwischen etwa 0,3 und 10 mg/kg basiert.

7. Zusammensetzung zur Verwendung nach einem der vorhergehenden Ansprüche, wobei zudem ein Wachstumsfaktor-Inhibitor verabreicht wird, wobei der Wachstumsfaktor-Inhibitor gegebenenfalls einen Rezeptor-TyrosinKinase (RTK)-Inhibitor, einen Src-Inhibitor, einen Anti-Metabolit-Inhibitor, ein Gemcitabin, ein GEMZAR^{™}, ein Mitosegift, ein Paclitaxel, ein Taxol, ein ABRAXANE^{™}, ein Erlotinib, ein TARCEVA^{™}, ein Lapatinib, ein TYKERB^{™}, ein Cetuxamib, ein ERBITUX^{™} oder einen Insulin-Wachstumsfaktor-Inhibitor umfasst.

8. Kit, das die pharmazeutische Zusammensetzung oder Rezeptur zur Verwendung nach einem der Ansprüche 1-7 umfasst.

## Revendications

1. Composition pharmaceutique ou formulation pour son utilisation pour détruire ou réduire le nombre de cellules cancéreuses ou de cellules tumorales résistant aux médicaments, ou de cellules souches cancéreuses (CSC) résistant aux médicaments, exprimant un polypeptide αvβ3 (avb3) chez un individu qui en a besoin, dans laquelle la composition pharmaceutique ou une formulation comprend :
un anticorps anti-intégrine αvβ3 qui peut se lier spécifiquement à un polypeptide d'intégrine αvβ3 (avb3) exprimé sur la cellule cancéreuse résistant aux médicaments ou la cellule tumorale résistant aux médicaments ou sur la CSC résistant aux médicaments, dans laquelle l'anticorps a un domaine Fc ou un domaine ou fraction équivalent(e) qui peut lier un macrophage et initier une destruction par cytotoxicité à médiation cellulaire dépendante des anticorps (ADCC) de la cellule à laquelle l'anticorps se lie spécifiquement, dans laquelle l'anticorps est l'anticorps monoclonal LM609 ou une version humanisée de celui-ci, et
dans laquelle les cellules cancéreuses, les cellules tumorales ou les cellules souches cancéreuses (CSC) résistant aux médicaments exprimant un polypeptide αvβ3 (avb3) sont résistantes à un inhibiteur de récepteur à activité tyrosine kinase (RTK).

2. Composition pour son utilisation selon la revendication 1, dans laquelle le macrophage est un macrophage humain ou un macrophage associé à une tumeur (TAM).

3. Composition pour son utilisation selon l'une quelconque des revendications précédentes, dans laquelle le cancer est un cancer épithélial ou une cellule tumorale épithéliale.

4. Composition pour son utilisation selon l'une quelconque des revendications précédentes, dans laquelle l'anticorps est administré par voie intraveineuse, par voie intramusculaire ou par voie sous-cutanée à l'individu qui en a besoin.

5. Composition pour son utilisation selon l'une quelconque des revendications précédentes, dans laquelle l'anticorps est formulé sous la forme d'une composition pharmaceutique ou d'une formulation stérile, ou est formulé pour une administration par voie intraveineuse, par voie intramusculaire ou par voie sous-cutanée.

6. Composition pour son utilisation selon l'une quelconque des revendications précédentes, dans laquelle le dosage de l'anticorps est basé soit sur un dosage fixe ou sur un dosage basé sur un poids corporel, ou sur une dose fixée comprise entre environ 100 et 1200 mg par mois, ou selon un dosage compris entre environ 0,3 et 10 mg/kg.

7. Composition pour son utilisation selon l'une quelconque des revendications précédentes, dans laquelle un inhibiteur du facteur de croissance est également administré, dans laquelle, facultativement, l'inhibiteur du facteur de croissance comprend un inhibiteur de récepteur à activité tyrosine kinase (RTK), un inhibiteur de Src, un inhibiteur anti-métabolite, une gemcitabine, un GEMZAR^{™}, un poison mitotique, un paclitaxel, un taxol, une ABRAXANE^{™}, un erlotinib, un TARCEVA^{™}, un lapatinib, un TYKERB^{™}, un cétuxamib, un ERBITUX^{™} ou un inhibiteur du facteur de croissance.

8. Kit comprenant la composition pharmaceutique ou la formulation pour son utilisation selon l'une quelconque des revendications 1 à 7.
